# EUROPEAN PATENT APPLICATION

(11) **EP 4 847 492 A1**
(43) Date of publication of application: **02.12.2026**
(21) Application number: 25744558.5
(22) Date of filing: 21.01.2025
(51) Int. Cl.: C07D 261/20, A61K 31/423, A61P 9/04

(54) **USE OF SALT FORMS OF COMPOUND AND CRYSTAL FORMS THEREOF IN TREATMENT OF HEART FAILURE WITH PRESERVED EJECTION FRACTION**

(30) Priority: 25.01.2024 CN 202410108651
(71) Applicant: Beijing Baheal Wisart Medical Research Co., Ltd, Beijing 102300 (CN)
(72) Inventor: WANG, Li, Beijing 100037 (CN); LI, Zheng, Beijing 100037 (CN)
(74) Representative: Ghirardi, Valeria
(86) International application number: PCT/CN2025/073510
(87) International publication number: WO 2025/157117

(57) **Abstract**

The present invention relates to the use of salt forms of a compound and crystal forms thereof in the treatment of heart failure with preserved ejection fraction. Specifically provided are salts of the compound as shown in formula I, wherein the salts are sodium salts, potassium salts, calcium salts, tert-butylamine salts, ammonium salts, choline salts, L-lysine salts, diethylamine salts, dimethylethanolamine salts, betaine salts or ethylenediamine salts of the compound as shown in formula I.

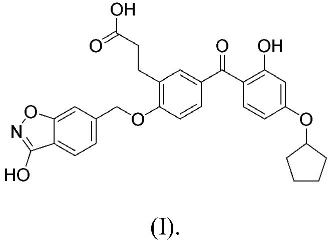

## Description

### Technical Field

The present invention relates to the field of medicine, specifically to the use of salt forms of a compound and crystal forms thereof in treatment of heart failure with preserved ejection fraction.

### Background Art

Cardiovascular disease represents one of the significant challenges in medicine. Heart failure (HF) is a major cause of death in patients with cardiovascular disease and a significant clinical challenge. Heart failure includes heart failure with reduced ejection fraction (HFrEF) and heart failure with preserved ejection fraction (HFpEF).

Currently, HFpEF accounts for approximately 50% of all heart failure patients, and its prevalence is growing at an astonishing rate. It is also a leading cause of the rising mortality rate of cardiovascular diseases. HFpEF is a complex condition involving multi-organ dysfunction, where the heart, lungs, kidneys, skeleton, immune system, inflammation, metabolism, and other components collectively contribute to the symptoms and outcomes. It is often accompanied by conditions such as obesity, hypertension, cardiac hypertrophy, diabetes, or atrial fibrillation. HFpEF is a syndrome with high morbidity and mortality. Clinical statistics show a mortality rate of 35% due to HF, with HFpEF accounting for 57% of these deaths. However, to date, very few drug therapies or medical devices have been proven to alter the disease progression and prognosis of HFpEF patients. There is an urgent need in the art to develop effective drugs and/or treatment methods for HFpEF.

*Jun* is a transcription factor, a member of the AP1 family, possessing chromatin-binding activity and transcription cis-regulatory region-binding activity. It is involved in processes including animal organ development, protein phosphorylation, and cell proliferation. A *Jun* inhibitor refers to an inhibitor that can suppress the expression of the *Jun* gene, reduce the binding activity of *Jun* to DNA, reduce the level of the *Jun* gene expression product, or prevent or block the signal transduction of *Jun*. Studies have shown that *Jun* inhibitors have therapeutic effects in animal models of endometriosis, breast cancer, sepsis, etc.

### Contents of the Invention

The inventors of the present invention have discovered for the first time that using a *Jun* inhibitor (e.g., T-5224) to inhibit the high expression of *Jun* can prevent and treat HFpEF. Based on this discovery, the inventors further screened salt forms of T-5224 and crystal forms thereof and obtained salt forms and crystal forms thereof with high stability, good solubility, high bioavailability, and excellent therapeutic efficacy against HFpEF, which have high application value.

To this end, in a first aspect, the present invention provides a salt of a compound represented by Formula I, wherein the salt is a sodium salt, potassium salt, calcium salt, tert-butylamine salt, ammonium salt, choline salt, L-lysine salt, diethylamine salt, dimethylethanolamine salt, betaine salt, or ethylenediamine salt of the compound represented by Formula I,

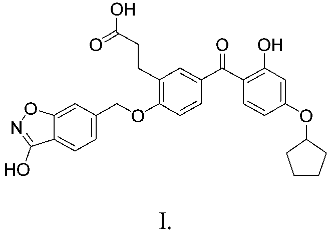

In some embodiments, the salt is a sodium salt, potassium salt, tert-butylamine salt, choline salt, L-lysine salt, diethylamine salt, dimethylethanolamine salt, betaine salt, or ethylenediamine salt of the compound represented by Formula I.

In some embodiments, the salt is a choline salt, diethylamine salt, or betaine salt of the compound represented by Formula I.

In some embodiments, the salt is a choline salt or betaine salt of the compound represented by Formula I.

In some embodiments, the salt is a choline salt of the compound represented by Formula I.

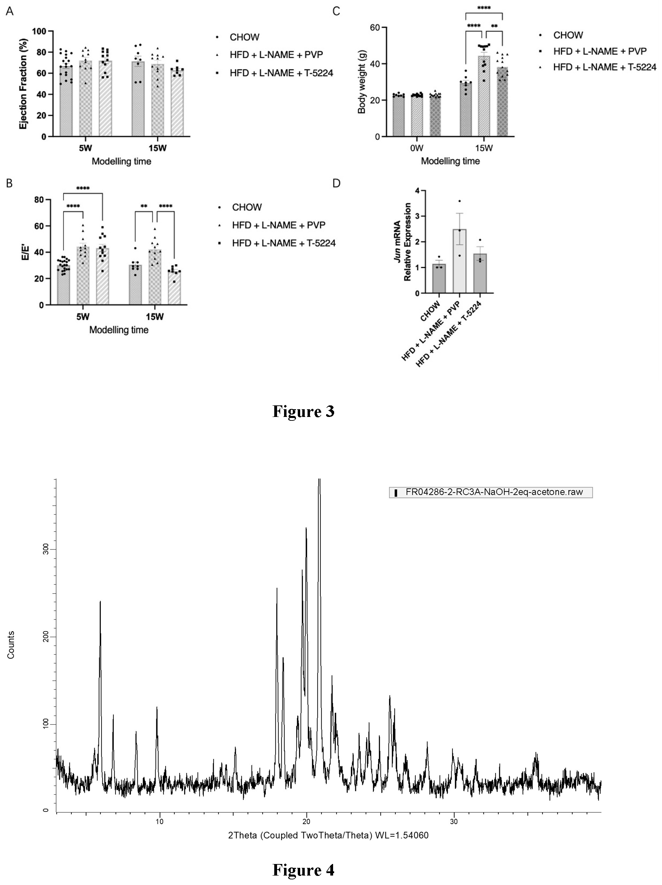

In some embodiments, the sodium salt of the compound represented by Formula I is in crystal Form A, wherein the crystal Form A of the sodium salt of the compound represented by Formula I has an X-ray powder diffraction pattern substantially the same as that shown in Figure 4.

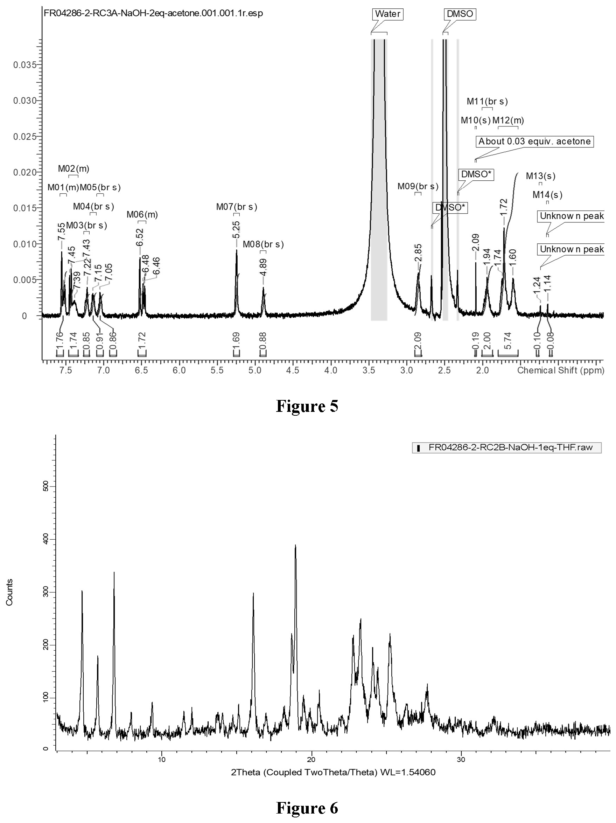

In some embodiments, the sodium salt of the compound represented by Formula I is in crystal Form B, wherein the crystal Form B of the sodium salt of the compound represented by Formula I has an X-ray powder diffraction pattern substantially the same as that shown in Figure 6.

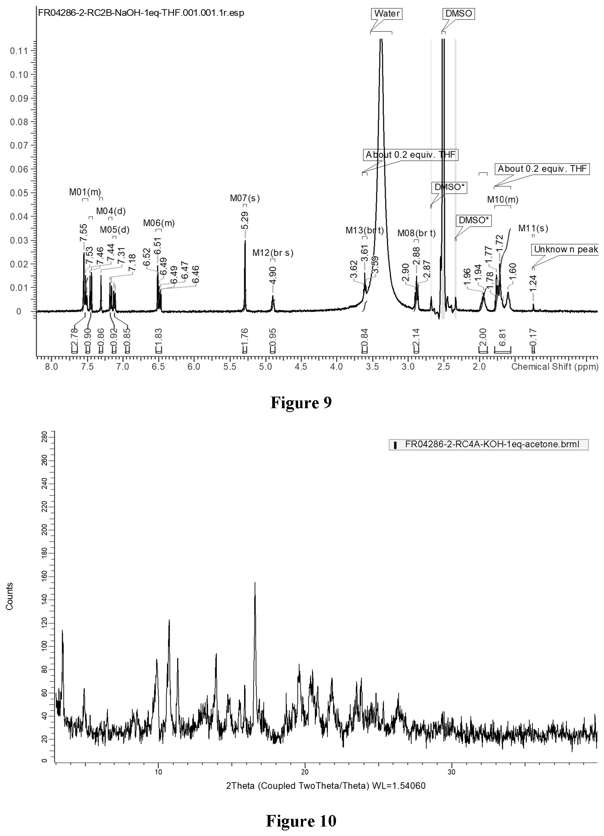

In some embodiments, the potassium salt of the compound represented by Formula I is in crystal Form A, wherein the crystal Form A of the potassium salt of the compound represented by Formula I has an X-ray powder diffraction pattern substantially the same as that shown in Figure 10.

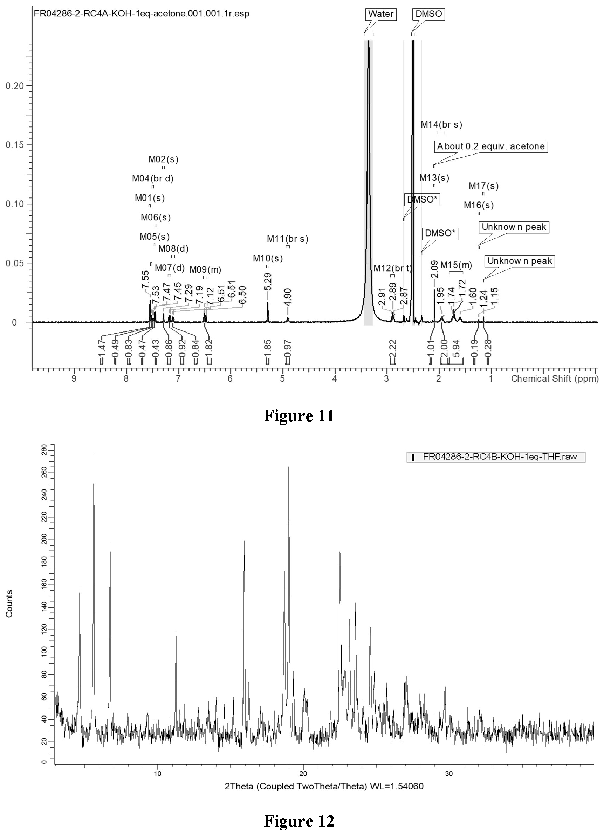

In some embodiments, the potassium salt of the compound represented by Formula I is in crystal Form B, wherein the crystal Form B of the potassium salt of the compound represented by Formula I has an X-ray powder diffraction pattern substantially the same as that shown in Figure 12.

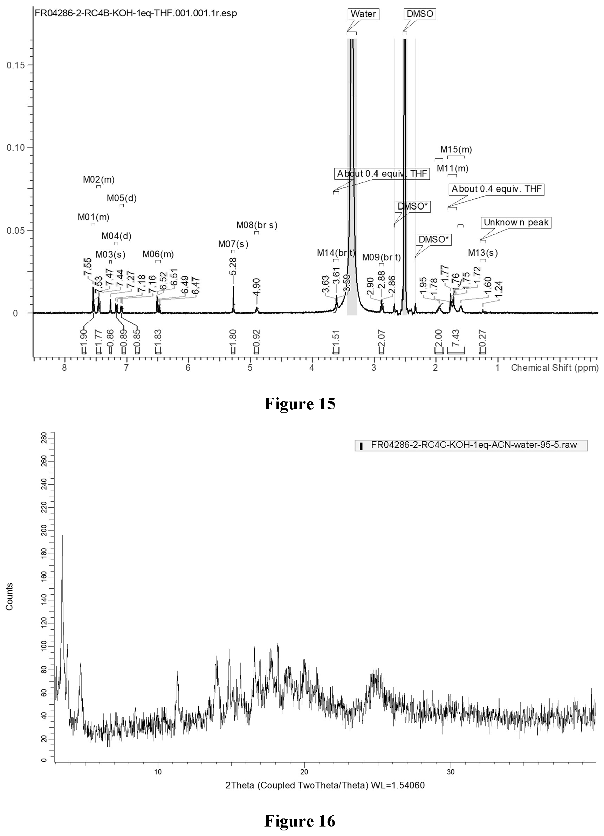

In some embodiments, the potassium salt of the compound represented by Formula I is in crystal Form C, wherein the crystal Form C of the potassium salt of the compound represented by Formula I has an X-ray powder diffraction pattern substantially the same as that shown in Figure 16.

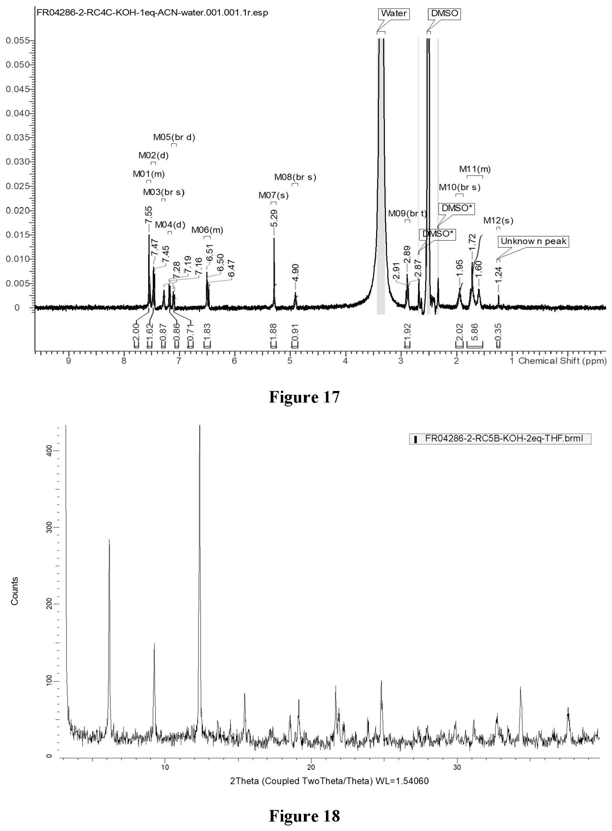

In some embodiments, the potassium salt of the compound represented by Formula I is in crystal Form D, wherein the crystal Form D of the potassium salt of the compound represented by Formula I has an X-ray powder diffraction pattern substantially the same as that shown in Figure 18.

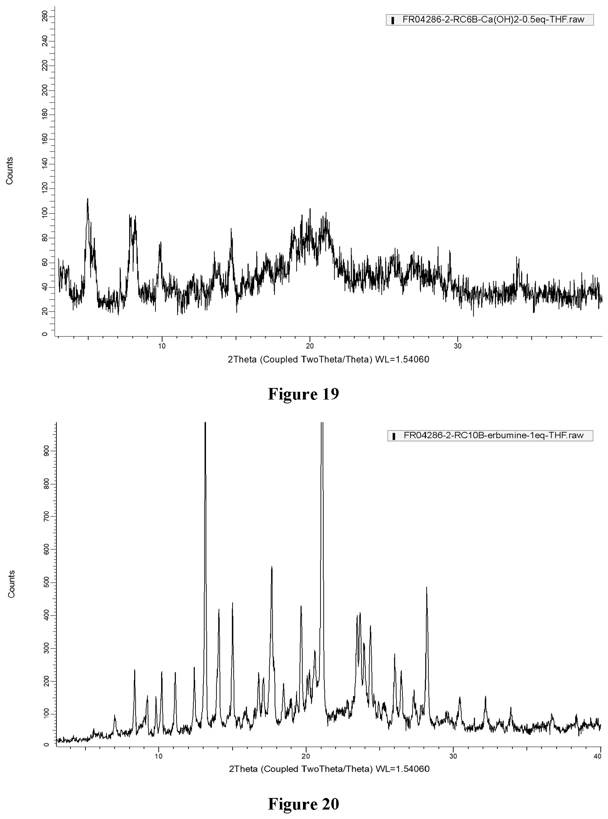

In some embodiments, the calcium salt of the compound represented by Formula I is in crystal Form A, wherein the crystal Form A of the calcium salt of the compound represented by Formula I has an X-ray powder diffraction pattern substantially the same as that shown in Figure 19.

In some embodiments, the tert-butylamine salt of the compound represented by Formula I is in crystal Form A, wherein the crystal Form A of the tert-butylamine salt of the compound represented by Formula I has an X-ray powder diffraction pattern substantially the same as that shown in Figure 20.

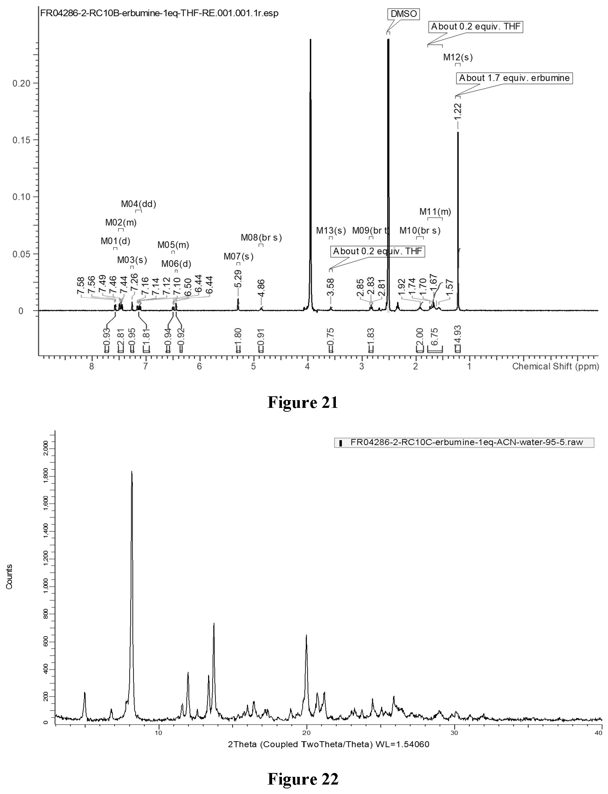

In some embodiments, the tert-butylamine salt of the compound represented by Formula I is in crystal Form B, wherein the crystal Form B of the tert-butylamine salt of the compound represented by Formula I has an X-ray powder diffraction pattern substantially the same as that shown in Figure 22.

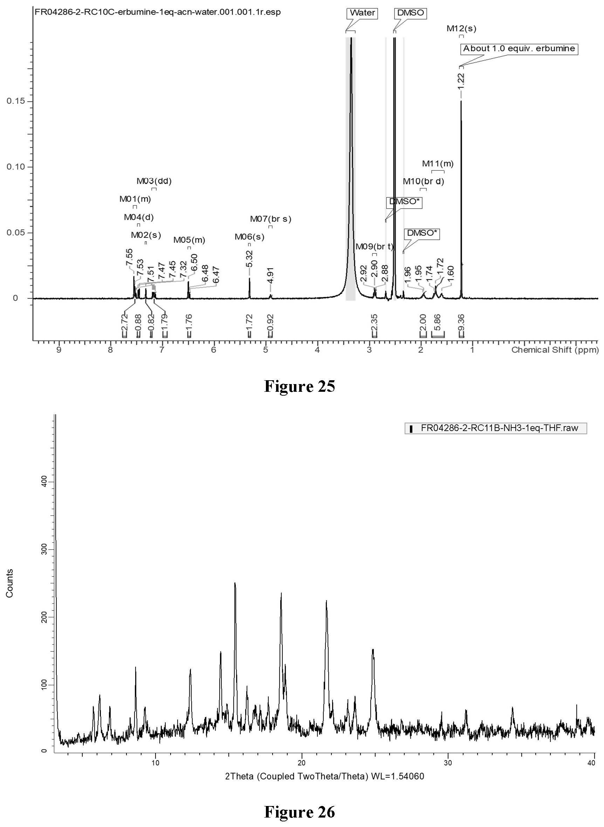

In some embodiments, the ammonium salt of the compound represented by Formula I is in crystal Form A, wherein the crystal Form A of the ammonium salt of the compound represented by Formula I has an X-ray powder diffraction pattern substantially the same as that shown in Figure 26.

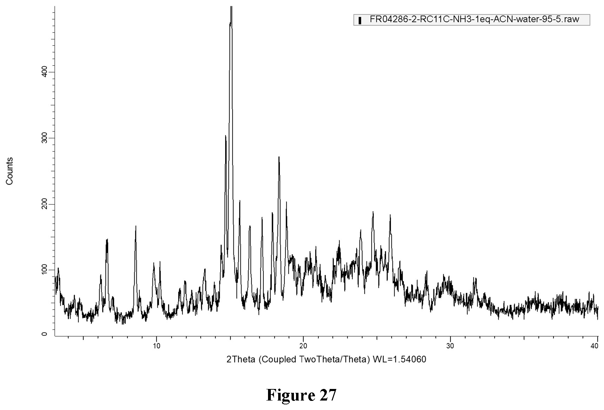

In some embodiments, the ammonium salt of the compound represented by Formula I is in crystal Form B, wherein the crystal Form B of the ammonium salt of the compound represented by Formula I has an X-ray powder diffraction pattern substantially the same as that shown in Figure 27.

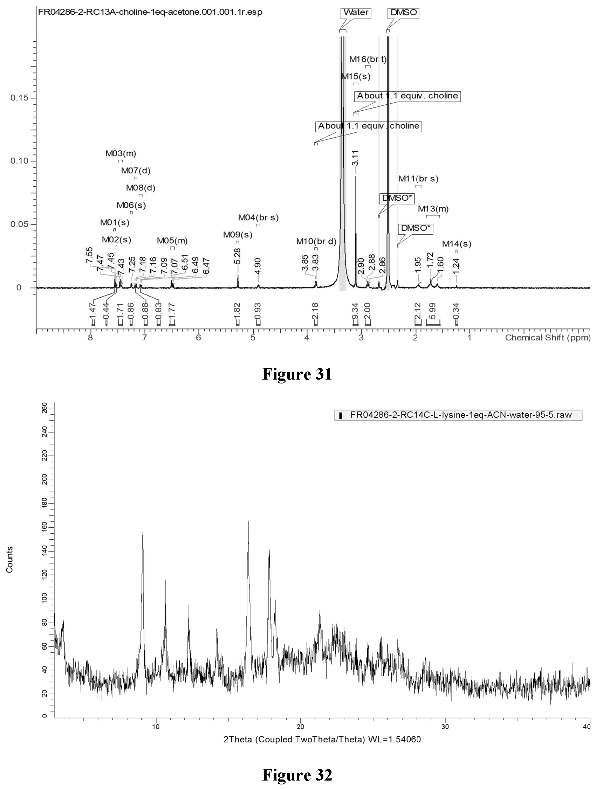

In some embodiments, the L-lysine salt of the compound represented by Formula I is in crystal Form A, wherein the crystal Form A of the L-lysine salt of the compound represented by Formula I has an X-ray powder diffraction pattern substantially the same as that shown in Figure 32.

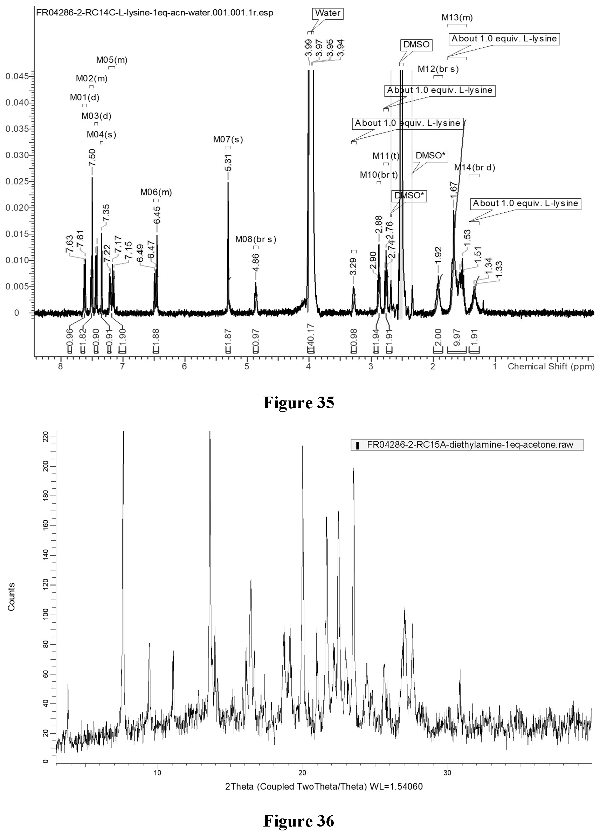

In some embodiments, the diethylamine salt of the compound represented by Formula I is in crystal Form A, wherein the crystal Form A of the diethylamine salt of the compound represented by Formula I has an X-ray powder diffraction pattern substantially the same as that shown in Figure 36.

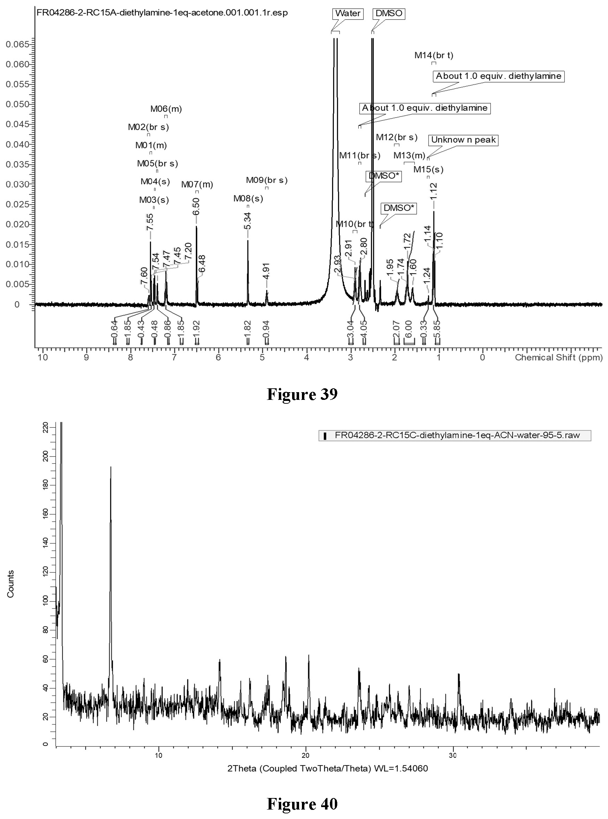

In some embodiments, the diethylamine salt of the compound represented by Formula I is in crystal Form B, wherein the crystal Form B of the diethylamine salt of the compound represented by Formula I has an X-ray powder diffraction pattern substantially the same as that shown in Figure 40.

In some embodiments, the diethylamine salt of the compound represented by Formula I is in crystal Form C, wherein the crystal Form C of the diethylamine salt of the compound represented by Formula I has characteristic peaks in its X-ray powder diffraction pattern at 2θ angles of 4.7±0.2°, 6.4±0.2°, 9.4±0.2°, 13.8±0.2°, 15.0±0.2°, 16.8±0.2°, 19.7±0.2°, and 20.4±0.2°.

In some embodiments, the crystal Form C of the diethylamine salt of the compound represented by Formula I has characteristic peaks in its X-ray powder diffraction pattern at 2θ angles of 4.7±0.2°, 6.4±0.2°, 9.4±0.2°, 13.1±0.2°, 13.8±0.2°, 14.1±0.2°, 15.0±0.2°, 16.8±0.2°, 18.0±0.2°, 18.9±0.2°, 19.4±0.2°, 19.7±0.2°, 20.4±0.2°, 23.0±0.2°, 23.3±0.2°, and 24.9±0.2°.

In some embodiments, the crystal Form C of the diethylamine salt of the compound represented by Formula I has an X-ray powder diffraction pattern with 2θ, d, and peak intensity values as shown in Table C.

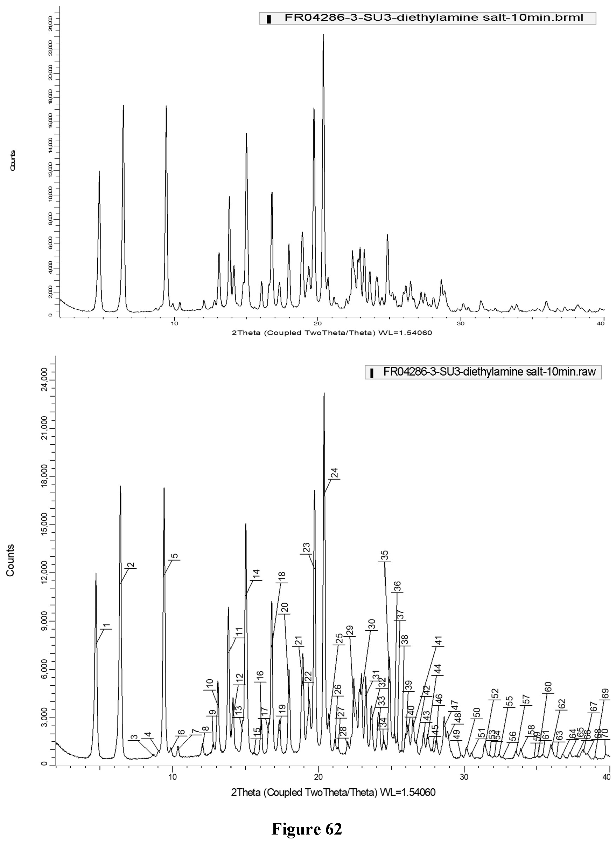

In some embodiments, the crystal Form C of the diethylamine salt of the compound represented by Formula I has an X-ray powder diffraction pattern substantially the same as that shown in Figure 62.

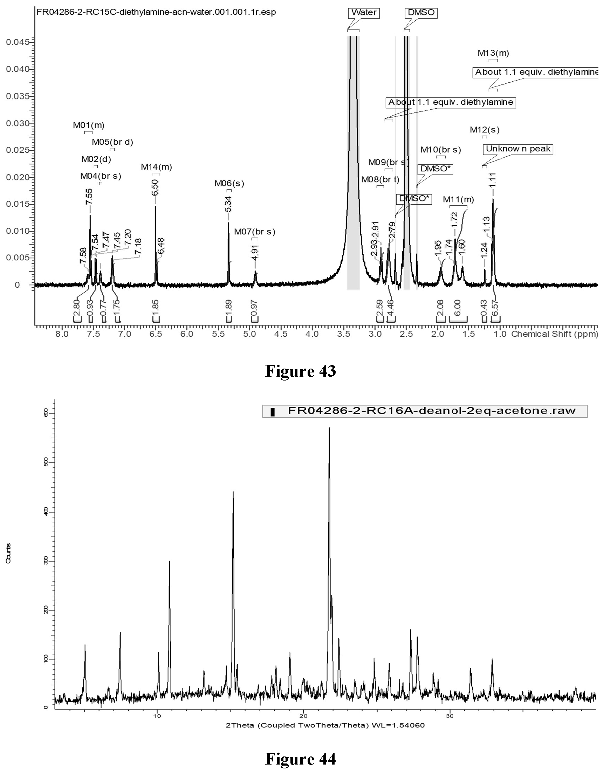

In some embodiments, the dimethylethanolamine salt of the compound represented by Formula I is in crystal Form A, wherein the crystal Form A of the dimethylethanolamine salt of the compound represented by Formula I has an X-ray powder diffraction pattern substantially the same as that shown in Figure 44.

In some embodiments, the betaine salt of the compound represented by Formula I is in crystal Form A, wherein the crystal Form A of the betaine salt of the compound represented by Formula I has characteristic peaks in its X-ray powder diffraction pattern at 2θ angles of 8.7±0.2°, 11.8±0.2°, 15.4±0.2°, 16.3±0.2°, 19.6±0.2°, 21.4±0.2°, and 23.4±0.2°.

In some embodiments, the crystal Form A of the betaine salt of the compound represented by Formula I has characteristic peaks in its X-ray powder diffraction pattern at 2θ angles of 8.1±0.2°, 8.7±0.2°, 11.8±0.2°, 15.4±0.2°, 16.3±0.2°, 18.4±0.2°, 19.6±0.2°, 20.4±0.2°, 21.4±0.2°, 22.3±0.2°, 23.4±0.2°, 24.2±0.2°, and 25.9±0.2°.

In some embodiments, the crystal Form A of the betaine salt of the compound represented by Formula I has an X-ray powder diffraction pattern with 2θ, d, and peak intensity values as shown in Table A.

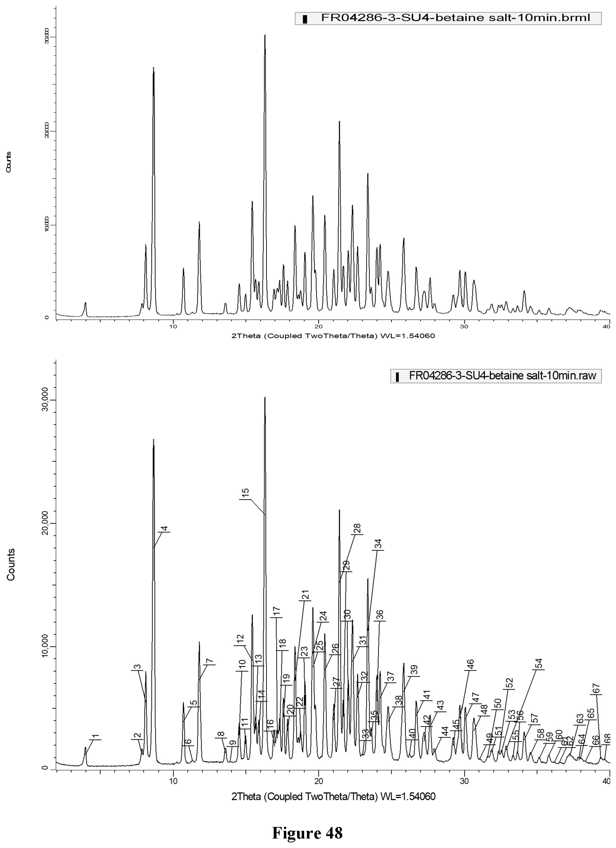

In some embodiments, the crystal Form A of the betaine salt of the compound represented by Formula I has an X-ray powder diffraction pattern substantially the same as that shown in Figure 48.

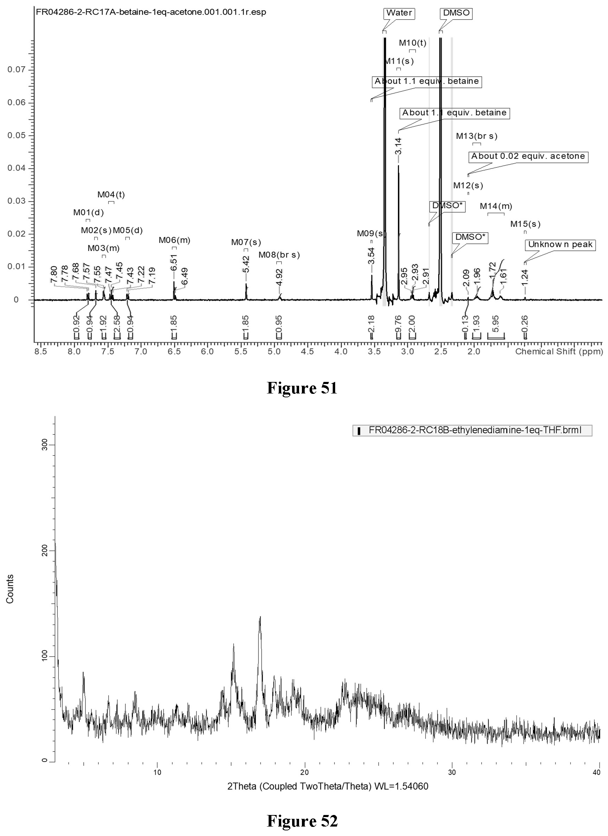

In some embodiments, the ethylenediamine salt of the compound represented by Formula I is in crystal Form A, wherein the crystal Form A of the ethylenediamine salt of the compound represented by Formula I has an X-ray powder diffraction pattern substantially the same as that shown in Figure 52.

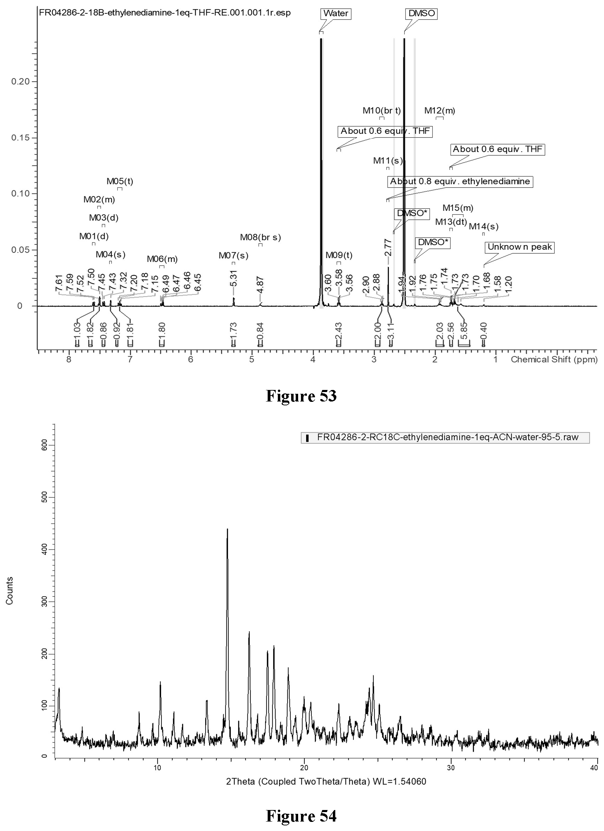

In some embodiments, the ethylenediamine salt of the compound represented by Formula I is in crystal Form B, wherein the crystal Form B of the ethylenediamine salt of the compound represented by Formula I has an X-ray powder diffraction pattern substantially the same as that shown in Figure 54.

In some embodiments, the choline salt of the compound represented by Formula I is in crystal Form C, wherein the crystal Form C of the choline salt of the compound represented by Formula I has characteristic peaks in its X-ray powder diffraction pattern at 2θ angles of 4.7±0.2°, 6.4±0.2°, 9.4±0.2°, 15.0±0.2°, 16.8±0.2°, 19.7±0.2°, and 20.4±0.2°.

In some embodiments, the crystal Form C of the choline salt of the compound represented by Formula I has characteristic peaks in its X-ray powder diffraction pattern at 2θ angles of 4.7±0.2°, 6.4±0.2°, 9.4±0.2°, 13.1±0.2°, 13.8±0.2°, 15.0±0.2°, 16.8±0.2°, 18.9±0.2°, 19.7±0.2°, 20.4±0.2°, 22.4±0.2°, 22.9±0.2°, 23.2±0.2°, and 24.9±0.2°.

In some embodiments, the crystal Form C of the choline salt of the compound represented by Formula I has an X-ray powder diffraction pattern with 2θ, d, and peak intensity values as shown in Table E.

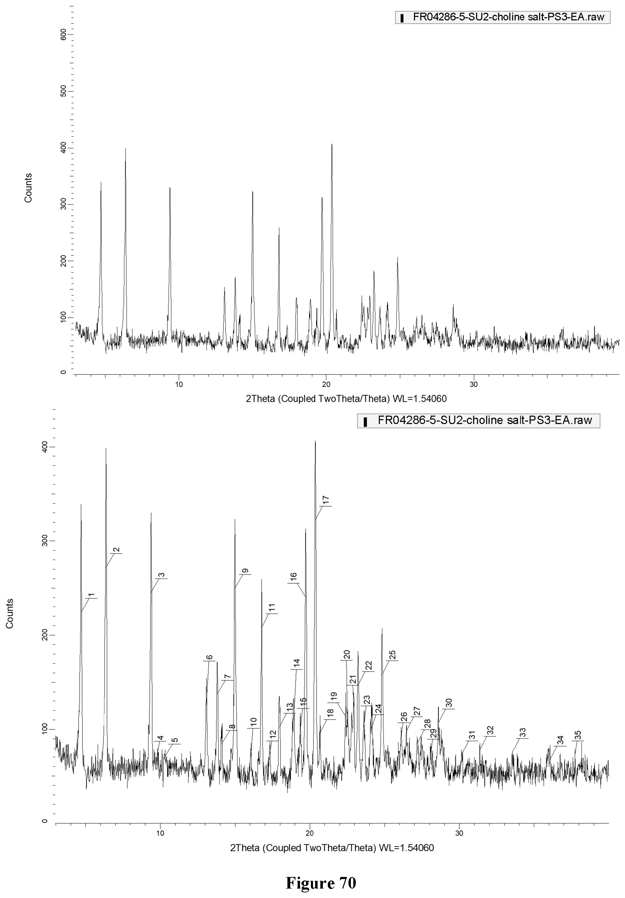

In some embodiments, the crystal Form C of the choline salt of the compound represented by Formula I has an X-ray powder diffraction pattern substantially the same as that shown in Figure 70.

In some embodiments, the choline salt of the compound represented by Formula I is in crystal Form A, wherein the crystal Form A of the choline salt of the compound represented by Formula I has characteristic peaks in its X-ray powder diffraction pattern at 2θ angles of 6.9±0.2°, 14.0±0.2°, 19.7±0.2°, 21.2±0.2°, 22.8±0.2°, 26.5±0.2°, and 27.0±0.2°.

In some embodiments, the crystal Form A of the choline salt of the compound represented by Formula I has characteristic peaks in its X-ray powder diffraction pattern at 2θ angles of 6.9±0.2°, 11.7±0.2°, 14.0±0.2°, 15.0±0.2°, 17.7±0.2°, 18.6±0.2°, 19.1±0.2°, 19.7±0.2°, 20.9±0.2°, 21.2±0.2°, 22.8±0.2°, 26.3±0.2°, 26.5±0.2°, 27.0±0.2°, and 27.6±0.2°.

In some embodiments, the crystal Form A of the choline salt of the compound represented by Formula I has an X-ray powder diffraction pattern with 2θ, d, and peak intensity values as shown in Table B.

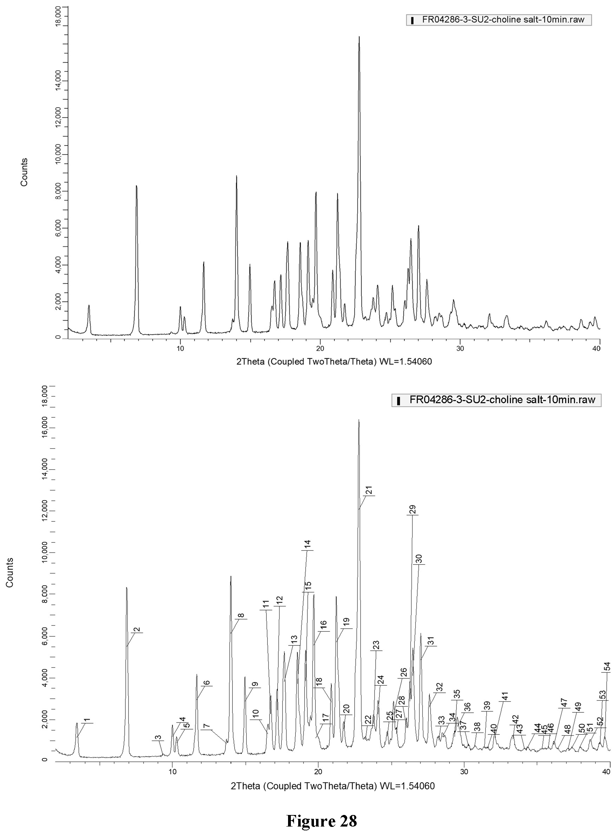

In some embodiments, the crystal Form A of the choline salt of the compound represented by Formula I has an X-ray powder diffraction pattern substantially the same as that shown in Figure 28.

In some embodiments, the choline salt of the compound represented by Formula I is in crystal Form B, wherein the crystal Form B of the choline salt of the compound represented by Formula I has characteristic peaks in its X-ray powder diffraction pattern at 2θ angles of 8.9±0.2°, 17.0±0.2°, 17.4±0.2°, 19.5±0.2°, 20.4±0.2°, 22.2±0.2°, 22.7±0.2°, and 27.2±0.2°.

In some embodiments, the crystal Form B of the choline salt of the compound represented by Formula I has characteristic peaks in its X-ray powder diffraction pattern at 2θ angles of 8.9±0.2°, 16.2±0.2°, 17.0±0.2°, 17.4±0.2°, 19.5±0.2°, 19.9±0.2°, 20.4±0.2°, 20.9±0.2°, 21.8±0.2°, 22.2±0.2°, 22.5±0.2°, 22.7±0.2°, 26.5±0.2°, 27.2±0.2°, and 27.5±0.2°.

In some embodiments, the crystal Form B of the choline salt of the compound represented by Formula I has an X-ray powder diffraction pattern with 2θ, d, and peak intensity values as shown in Table D.

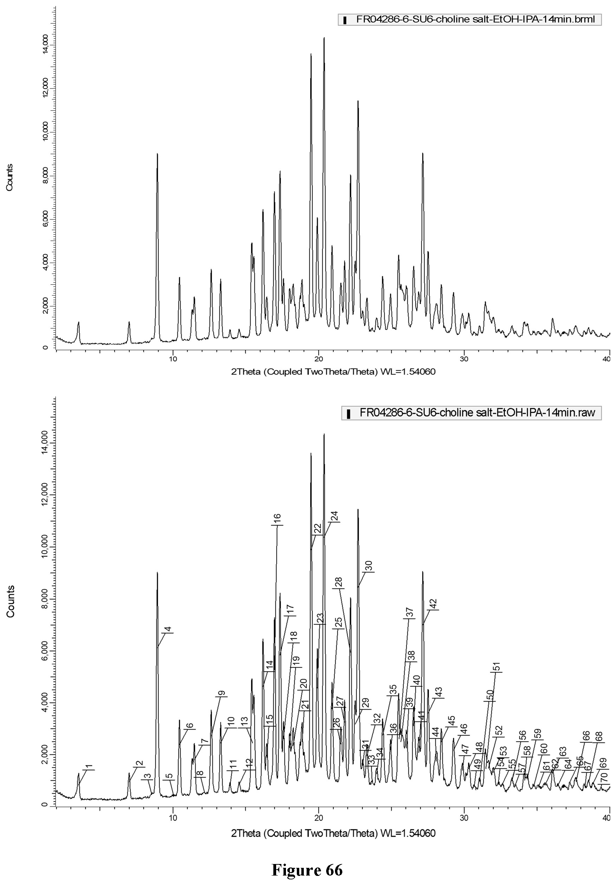

In some embodiments, the crystal Form B of the choline salt of the compound represented by Formula I has an X-ray powder diffraction pattern substantially the same as that shown in Figure 66.

In a second aspect, the present invention provides a pharmaceutical composition, comprising the salt as described above, and optionally further comprising a pharmaceutically acceptable excipient.

In a third aspect, the present invention provides use of the salt as described above in the manufacture of a medicament for treating and/or preventing heart failure with preserved ejection fraction;
or, provides the salt as described above for use in treating and/or preventing heart failure with preserved ejection fraction;
or, provides a method for treating and/or preventing heart failure with preserved ejection fraction, comprising administering an effective amount of the salt as described above to a subject in need thereof.

### Brief Description of the Drawings

Figure 1 shows the construction of the HFpEF model. A: Schematic diagram of the experimental procedure; B: Detection results of systolic function in mice after 5 weeks of feeding; C: Detection results of diastolic function in mice after 5 weeks of feeding.
Figure 2 shows the relative expression of *Jun* in cardiomyocytes after 15 weeks of HFD + L-NAME feeding, indicating high expression of *Jun* in HFpEF model mice.
Figure 3 shows that T-5224 effectively alleviated the occurrence and development of HFpEF. A: Detection results of systolic function in mice at different time points; B: Detection results of diastolic function in mice at different time points; C: Changes in body weight of mice under different treatments; D: Changes in *Jun* expression levels in mouse cardiomyocytes under different treatments.
Figure 4 shows the XRPD pattern of T-5224 sodium salt Form A.
Figure 5 shows the ¹H-NMR spectrum of T-5224 sodium salt Form A.
Figure 6 shows the XRPD pattern of T-5224 sodium salt Form B.
Figure 7 shows the DSC thermogram of T-5224 sodium salt Form B.
Figure 8 shows the TGA thermogram of T-5224 sodium salt Form B.
Figure 9 shows the ¹H-NMR spectrum of T-5224 sodium salt Form B.
Figure 10 shows the XRPD pattern of T-5224 potassium salt Form A.
Figure 11 shows the ¹H-NMR spectrum of T-5224 potassium salt Form A.
Figure 12 shows the XRPD pattern of T-5224 potassium salt Form B.
Figure 13 shows the DSC thermogram of T-5224 potassium salt Form B.
Figure 14 shows the TGA thermogram of T-5224 potassium salt Form B.
Figure 15 shows the ¹H-NMR spectrum of T-5224 potassium salt Form B.
Figure 16 shows the XRPD pattern of T-5224 potassium salt Form C.
Figure 17 shows the ¹H-NMR spectrum of T-5224 potassium salt Form C.
Figure 18 shows the XRPD pattern of T-5224 potassium salt Form D.
Figure 19 shows the XRPD pattern of T-5224 calcium salt Form A.
Figure 20 shows the XRPD pattern of T-5224 tert-butylamine salt Form A.
Figure 21 shows the ¹H-NMR spectrum of T-5224 tert-butylamine salt Form A.
Figure 22 shows the XRPD pattern of T-5224 tert-butylamine salt Form B.
Figure 23 shows the DSC thermogram of T-5224 tert-butylamine salt Form B.
Figure 24 shows the TGA thermogram of T-5224 tert-butylamine salt Form B.
Figure 25 shows the ¹H-NMR spectrum of T-5224 tert-butylamine salt Form B.
Figure 26 shows the XRPD pattern of T-5224 ammonium salt Form A.
Figure 27 shows the XRPD pattern of T-5224 ammonium salt Form B.
Figure 28 shows the XRPD pattern of T-5224 choline salt Form A.
Figure 29 shows the DSC thermogram of T-5224 choline salt Form A.
Figure 30 shows the TGA thermogram of T-5224 choline salt Form A.
Figure 31 shows the ¹H-NMR spectrum of T-5224 choline salt Form A.
Figure 32 shows the XRPD pattern of T-5224 L-lysine salt Form A.
Figure 33 shows the DSC thermogram of T-5224 L-lysine salt Form A.
Figure 34 shows the TGA thermogram of T-5224 L-lysine salt Form A.
Figure 35 shows the ¹H-NMR spectrum of T-5224 L-lysine salt Form A.
Figure 36 shows the XRPD pattern of T-5224 diethylamine salt Form A.
Figure 37 shows the DSC thermogram of T-5224 diethylamine salt Form A.
Figure 38 shows the TGA thermogram of T-5224 diethylamine salt Form A.
Figure 39 shows the ¹H-NMR spectrum of T-5224 diethylamine salt Form A.
Figure 40 shows the XRPD pattern of T-5224 diethylamine salt Form B.
Figure 41 shows the DSC thermogram of T-5224 diethylamine salt Form B.
Figure 42 shows the TGA thermogram of T-5224 diethylamine salt Form B.
Figure 43 shows the ¹H-NMR spectrum of T-5224 diethylamine salt Form B.
Figure 44 shows the XRPD pattern of T-5224 dimethylethanolamine salt Form A.
Figure 45 shows the DSC thermogram of T-5224 dimethylethanolamine salt Form A.
Figure 46 shows the TGA thermogram of T-5224 dimethylethanolamine salt Form A.
Figure 47 shows the ¹H-NMR spectrum of T-5224 dimethylethanolamine salt Form A.
Figure 48 shows the XRPD pattern of T-5224 betaine salt Form A.
Figure 49 shows the DSC thermogram of T-5224 betaine salt Form A.
Figure 50 shows the TGA thermogram of T-5224 betaine salt Form A.
Figure 51 shows the ¹H-NMR spectrum of T-5224 betaine salt Form A.
Figure 52 shows the XRPD pattern of T-5224 ethylenediamine salt Form A.
Figure 53 shows the ¹H-NMR spectrum of T-5224 ethylenediamine salt Form A.
Figure 54 shows the XRPD pattern of T-5224 ethylenediamine salt Form B.
Figure 55 shows the DSC thermogram of T-5224 ethylenediamine salt Form B.
Figure 56 shows the TGA thermogram of T-5224 ethylenediamine salt Form B.
Figure 57 shows the ¹H-NMR spectrum of T-5224 ethylenediamine salt Form B.
Figure 58 shows the XRPD pattern of T-5224 Form A.
Figure 59 shows the DSC thermogram of T-5224 Form A.
Figure 60 shows the TGA thermogram of T-5224 Form A.
Figure 61 shows the ¹H-NMR spectrum of T-5224 Form A.
Figure 62 shows the XRPD pattern of T-5224 diethylamine salt Form C.
Figure 63 shows the DSC thermogram of T-5224 diethylamine salt Form C.
Figure 64 shows the TGA thermogram of T-5224 diethylamine salt Form C.
Figure 65 shows the ¹H-NMR spectrum of T-5224 diethylamine salt Form C.
Figure 66 shows the XRPD pattern of T-5224 choline salt Form B.
Figure 67 shows the DSC thermogram of T-5224 choline salt Form B.
Figure 68 shows the TGA thermogram of T-5224 choline salt Form B.
Figure 69 shows the ¹H-NMR spectrum of T-5224 choline salt Form B.
Figure 70 shows the XRPD pattern of T-5224 choline salt Form C.
Figure 71 shows the DSC thermogram of T-5224 choline salt Form C.
Figure 72 shows the ¹H-NMR spectrum of T-5224 choline salt Form C.
Figure 73 shows that T-5224 choline salt Form B effectively inhibited the occurrence and development of HFpEF.

### Specific Models for Carrying Out the Invention

It should be understood that the terms used herein are for the purpose of describing particular embodiments only and are not intended to be limiting. Furthermore, although any methods, devices, and materials similar or equivalent to those described herein can be used in the practice or testing of the present invention, the preferred methods, devices, and materials are now described.

In the present invention, the term "treatment" generally refers to obtaining a desired pharmacological and/or physiological effect. The effect may be prophylactic in terms of completely or partially preventing a disease or its symptoms; and/or therapeutic in terms of partially or completely stabilizing or curing a disease and/or adverse effects caused by the disease. "Treatment" as used herein covers any treatment of a disease in a patient, including: (a) preventing the disease or symptoms in a patient who may be predisposed to the disease or symptom but has not yet been diagnosed; (b) inhibiting the symptoms of the disease, i.e., arresting its development; or (c) relieving the symptoms of the disease, i.e., causing regression of the disease or symptoms.

In the present invention, "subject" refers to a vertebrate. In certain embodiments, the vertebrate refers to a mammal. Mammals include, but are not limited to, livestock (such as cattle), pets (such as cats, dogs, and horses), primates, mice, and rats. In certain embodiments, the mammal refers to a human.

In the present invention, "effective amount" refers to an amount effective to achieve the desired therapeutic effect at the necessary dosage and for the necessary time. A "therapeutically effective amount" of a substance/molecule of the present invention may vary depending on factors such as the individual's disease state, age, sex, and weight, and the ability of the substance/molecule to elicit a desired response in the individual. A therapeutically effective amount also encompasses an amount where any toxic or detrimental effects of the substance/molecule are outweighed by the therapeutically beneficial effects.

The pharmaceutical composition of the present invention may comprise a pharmaceutically acceptable excipient, including but not limited to: ion exchanger, alumina, aluminum stearate, lecithin, serum protein such as human serum albumin, buffer substance such as phosphate, glycerin, sorbic acid, potassium sorbate, partial glyceride mixture of saturated vegetable fatty acids, water, salt or electrolyte, such as protamine sulfate, disodium hydrogen phosphate, potassium hydrogen phosphate, sodium chloride, zinc salt, colloidal silica, magnesium trisilicate, polyvinylpyrrolidone, cellulosic substance, polyethylene glycol, sodium carboxymethylcellulose, polyacrylate, beeswax, lanolin, and the like.

The pharmaceutical composition of the present invention can be prepared in various forms according to different routes of administration. For example, the pharmaceutical composition can be administered in any of the following manners: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal, and intracranial injection or infusion, or administration using an implanted depot device. Among them, oral or intravenous administration is preferred.

The compound of the present invention may optionally be used in combination with one or more other active ingredients, and the respective dosages and ratios thereof can be adjusted by those skilled in the art according to the specific disease, the patient's specific condition, clinical needs, etc.

The testing methods involved in the present invention are all well-known and commonly used in the art, for example as follows:

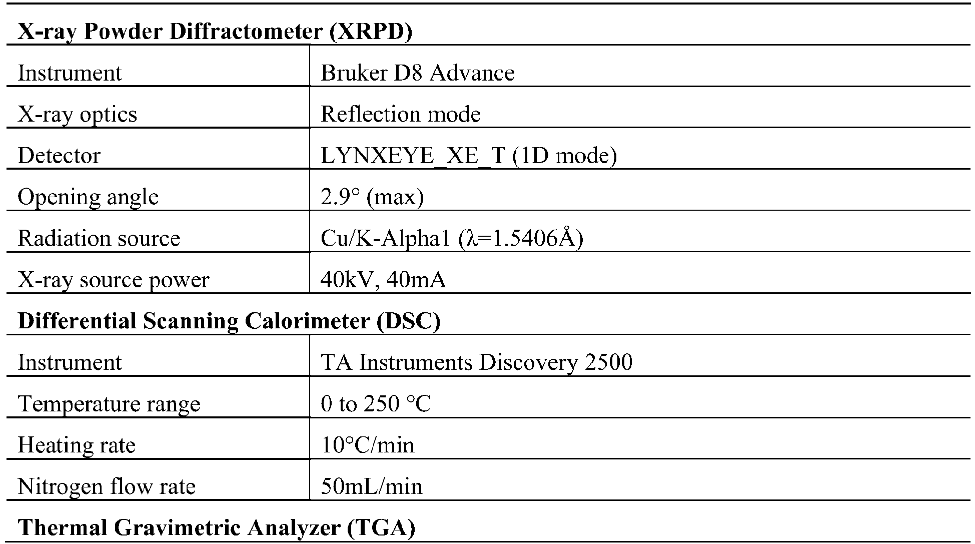

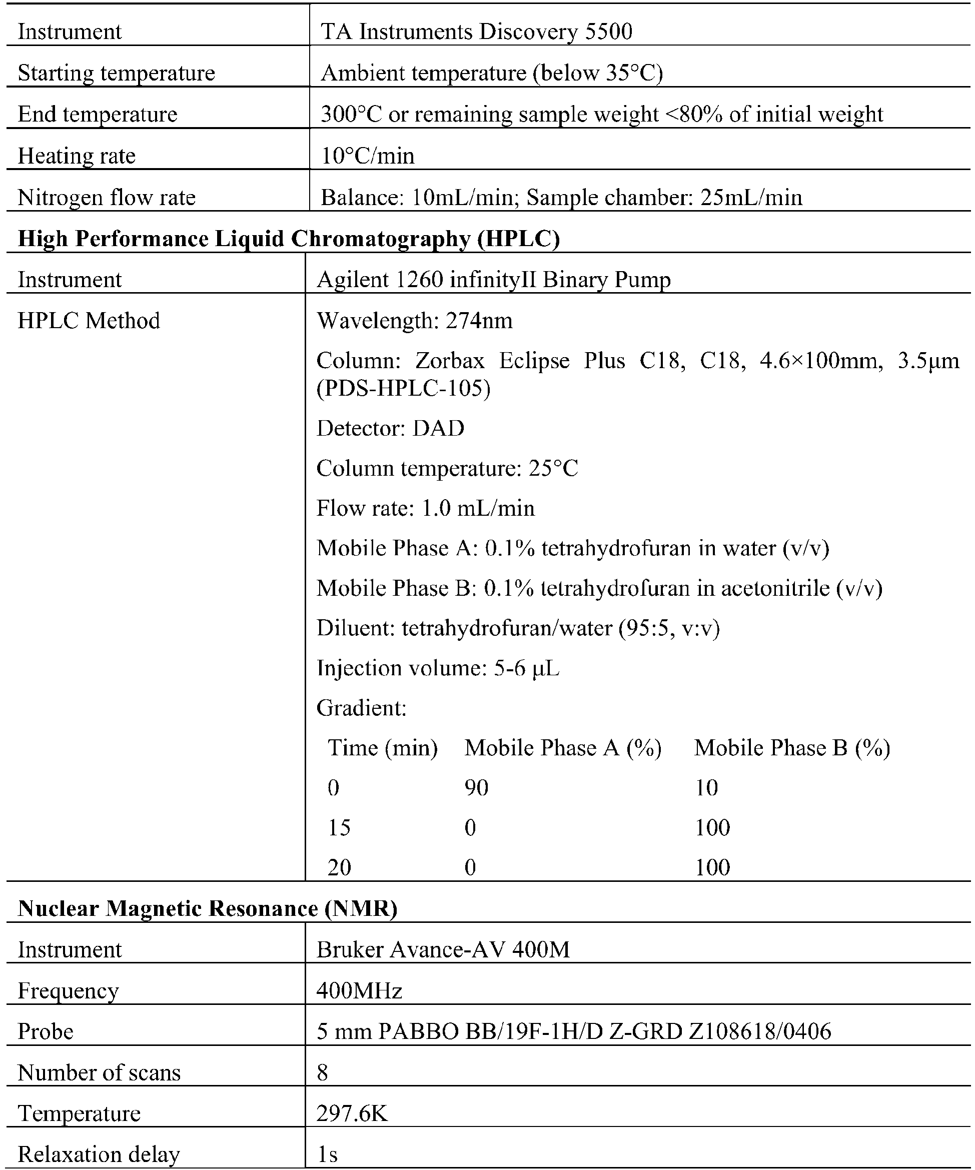

The present invention is further explained below with reference to specific examples.

### Example A: Preliminary Studies

### 1. Materials and reagents

In this example, C57BL/6N wild-type mice were purchased from Beijing Vital River. The sources of reagents were shown in the following table.

| Reagent | Cat. No. | Supplier |
|---|---|---|
| Collagenase type II | 17101015 | Gibico |
| T-5224 | S8966 | Selleck |
| Polyvinylpyrrolidone (PVP) | S3596 | Selleck |
| GeneJet RNA purification kit | K0732 | Thermo Scientific |
| iScript TM cDNA synthesis kit | 1708890 | Bio-Rad |
| iTaqUniversl SYBR Green supermix | 1725121 | Bio-Rad |

In addition to the above, other materials and reagents used in this example were also commercially available products.

### 2. Animal experiment guideline

In this example, all animal studies were conducted under the guidance of the Laboratory Animal Center of the Institutional Animal Care and Use Committee of Fuwai Hospital, National Center for Cardiovascular Diseases, China. All mice were propagated and raised in the same environment, and the mice were randomly divided into groups during the experiment. Echocardiographic analysis was performed by an independent researcher who was unaware of the study objectives.

### 3. Induction of model of heart failure with preserved ejection fraction

Male C57BL/6N wild-type mice aged 8 to 10 weeks were divided into three groups, namely, normal group (normal diet and drinking water), model control group (high-fat diet combined with administration of N-nitro-L-arginine methyl ester) and model treatment group (high-fat diet combined with administration of N-nitro-L-arginine methyl ester and treatment with T-5224). Among them, the model control group and the model treatment group were modeled by the following literature: Gabriele G. Schiattarella et al., Nitrosative stress drives heart failure with preserved ejection fraction, https://doi.org/10.1038/s41586-019-1100-z. Specifically, heart failure with preserved ejection fraction was induced by a high-fat diet (HFD) (60% kcal, from fat (lard)) and N-nitro-L-arginine methyl ester (abbreviated as L-NAME, 0.5 g/L in drinking water) to obtain the HFpEF animal model.

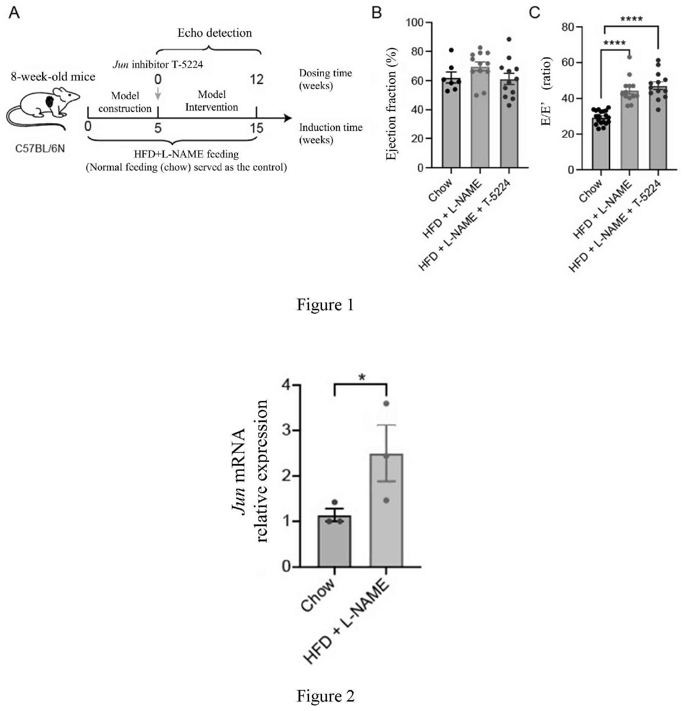

In the fifth week of model induction, the systolic function parameter LVEF of mice did not change, while the diastolic function parameter (E/E') increased significantly in the 5^{th} week of model induction, indicating that the model of heart failure with preserved ejection fraction recorded in the above literature had been successfully obtained. At the same time, there was no significant difference in the diastolic function parameter (E/E') between the model control group and the model treatment group in the 5^{th} week. Subsequent drug administration was carried out under the same baseline, as shown in Figure 1.

### 4. Jun expression was correlated with HFpEF

In the 15^{th} week of model induction, the cardiomyocytes of normal group and the model control group were isolated by perfusion method, and quantitative RCR detection was performed. The specific operations were as follows:

### 4.1. Isolation of adult mouse cardiomyocytes:

In order to isolate cardiomyocytes from the heart of adult mice, we used the classic perfusion method to isolate cardiomyocytes. Specifically, mice were injected with 100 µl of sodium heparin (1000 units in 50 ml) 20 minutes before being killed to prevent heart coagulation during the operation, which increased the difficulty of digestion. After that, the mice were anesthetized and killed, and the hearts were removed and transferred to a calcium-free solution for washing. Then, the Langendorff method was used for digestion. The heart was perfused with a calcium-free solution for 5 minutes using a Langendorff apparatus, and then digested with digestive enzyme solution (0.7 mg/ml type II collagenase and 0.7 mg/ml bovine serum albumin in a calcium-free solution) for about 30 minutes. The heart was constantly touched at about 20 minutes. When the heart became soft and slippery, which indicated that the digestion was basically completed, the tissues from the ventricles were then collected, cut into pieces, and gently pipetted to dissociate into single cells. The cells were allowed to settle, and the supernatant was taken to remove the undigested and adherent tissues, and centrifuged at 100 g and 4°C for 2 minutes to obtain cardiomyocyte pellets, and the supernatant was mostly non-cardiomyocytes. Cardiomyocytes were resuspended in a calcium-free solution containing 10% FBS for subsequent experiments. Non-cardiomyocytes could be resuspended with culture medium or PBS for subsequent experiments. If purer cardiomyocytes and non-cardiomyocytes were required, the cell suspension could be centrifuged (100g, 2 minutes at room temperature) three times to separate cardiomyocytes from non-cardiomyocytes. Cardiomyocytes were collected for further experiments.

### 4.2. Quantitative PCR detection:

Total RNA was extracted from cells using GeneJet RNA purification kit (Thermo Scientific, K0732), and 0.1µg of total RNA was reverse transcribed using iScript TM cDNA synthesis kit (Bio-Rad, 1708890) to generate cDNA. qPCR was performed using iTaqUniversl SYBR Green supermix (1725121, Bio-Rad) on ABI Vii7 real-time system (Life Technologies, Q6), and b-Actin was used for standardized quantitative analysis. As shown in Figure 2, compared with normal mice, high expression of *Jun* was significantly observed in the mouse HFpEF animal model. This indicated a correlation between *Jun* expression levels and HFpEF in mice, with *Jun* being highly expressed in HFpEF.

### 5. T-5224 administration method

After the animal model was obtained, that was, from the 5^{th} week of inducing the HFpEF model, the model treatment group was treated with T-5224, the model control group was treated with a solvent without drug, and the mice with normal diet and drinking water during the whole induction process were used as negative control. The mice were administrated with drugs when they were raised for 5 weeks. For the treatment group, T-5224 was administered at 250 mg/kg according to the body weight of mice. Specifically, the administration was performed every other day, 0.8 mg of T-5224 was dissolved in 200 uL of 1% PVP solution each time. The administration started from the 5^{th} week and ended at the 13^{th} week (a total of 15 administrations), with a total dose of 250 mg/kg. The control group was administrated with an equal volume of 1% PVP solution, and other treatments were the same.

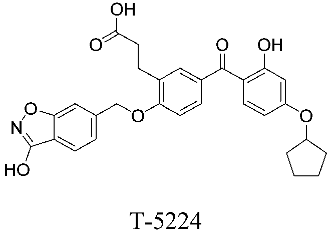

### 6. Conventional echocardiography and Doppler imaging

All the mice were raised under different conditions for five weeks and then began to undergo conventional echocardiography and Doppler imaging, which was performed every two weeks until the end of the 15^{th} week. Specifically, transthoracic echocardiography was performed using the VisualSonics Vevo 2100 system equipped with an MS400 transducer (Visual Sonics). Left ventricular ejection fraction (LVEF) and other systolic function indices were obtained from the short-axis M-mode scans at the midventricular level, as indicated by the presence of papillary muscles, in conscious, gently restrained mice. Cardiac apical four-chamber views were obtained in the anesthetized mice, and used for diastolic function measurements by pulsed waves and tissue Doppler imaging at the level of mitral valve. Anesthesia was induced by 2.5% isoflurane and confirmed by lack of response to firm pressure on one of the hind paws. During echocardiographic acquisition (under temperature-controlled conditions), isoflurane was reduced to 1.0% to 1.5% and adjusted to maintain heart rate in the range of 500 beats per minute. The parameters collected included: heart rate, left ventricular end-diastolic diameter, left ventricular end-systolic diameter, end-diastolic interventricular septal wall thickness, left ventricular end-diastolic posterior wall, left ventricular fractional shortening, LVEF, peak Doppler blood inflow velocity across mitral valve during early diastole, peak Doppler blood inflow velocity across mitral valve during late diastole, isovolumetric relaxation time, peak tissue Doppler of myocardial relaxation velocity at the mitral valve annulus during early diastole and early filling deceleration time. At the end of the procedure, all the mice recovered from anesthesia without any abnormalities. All parameters were measured at least 3 times, and the mean values were provided. The echocardiography and Doppler imaging included systolic function and diastolic function detection.

### 7. Experimental results and conclusions

Firstly, the detection of systolic function and diastolic function of mice in the 5^{th} week showed that there was no significant change in systolic function, but the diastolic function parameter E/E' increased significantly, proving that there was a disorder in diastolic function, indicating that the model described in the above literature was successfully obtained. At the same time, taking the 5^{th} week as the starting point of drug administration, there was no significant difference in cardiac diastolic function between the model control group and the model treatment group before drug administration (Figure 1A-C), and the drug administration was carried out on this basis.

Secondly, compared with the normal group, the expression of *Jun* in the model control group was upregulated, indicating that the expression of *Jun* was correlated with HFpEF. In the HFpEF mouse model, *Jun* was highly expressed (Figure 2). Based on this correlation, it could be judged that *Jun* inhibitor could be used for the prevention and treatment of HFpEF.

Further, T-5224 was used to verify the effectiveness of *Jun* inhibitor in preventing and treating HFpEF. After confirming that the model construction was successful and that the baselines of the model control group and the model treatment group were consistent, the model treatment group was treated with T-5224. The cardiac function detection results showed that in the model treatment group (after T-5224 administration), the occurrence and development of HFpEF had been well suppressed. Specifically, after treatment with T-5224, the diastolic function in the mice treated with high-fat diet combined with L-NAME (HFD + 0.5g/L L-NAME) was significantly improved and could be maintained until the 15^{th} week. However, in the model control mice that were not treated with T-5224, continued deterioration of diastolic function was observed (Figure 3A-B). At the same time, in the model treatment group, the expression of *Jun* was down-regulated compared with the model control group (Figure 3D), and the mouse obesity was improved (Figure 3C). This indicated that T-5224, as a *Jun* inhibitor, could bring about preventive and therapeutic effects on HFpEF in the mouse HFpEF model.

### Example 1: Screening and Preparation of T-5224 Salts

Based on the experimental results of Example A, the inventors further explored new salt forms of T-5224 and crystal forms thereof.

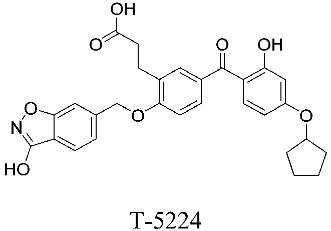

### 1. Suspension Method

Approximately 30 to 50 mg of Compound T-5224 Form A was weighed and placed into a 2 mL glass vial with 0.5, 1, or 2 equivalents of base. Screening solvents were added to conduct the screening experiment using the suspension method. The obtained samples were suspended at 50°C for 2 hours, then naturally cooled to 25°C, and suspended at 25°C for at least 48 hours.

The resulting suspension was centrifuged at 14,000 rpm through a 0.45 µm nylon filter. The obtained solid was vacuum dried at 50°C for 2 hours and characterized by XRPD.

**Table 1-1: Salt preparation by suspension method**

| **Base (equiv.)** | **Acetone** | **Tetrahydrofuran** | **Acetonitrile/Water (95:5, v:v)** |
|---|---|---|---|
| Sodium hydroxide (1.0 equiv.) | Sodium salt Form A | Sodium salt Form B | Sodium salt, low crystallinity sample |
| Sodium hydroxide (2.0 equiv.) | Sodium salt Form A | Gel-like substance | Gel-like substance |
| Potassium hydroxide (1.0 equiv.) | Potassium salt Form A | Potassium salt Form B | Potassium salt Form C |
| Potassium hydroxide (2.0 equiv.) | Potassium salt Form D | Potassium salt Form D | Yellow gel-like substance |
| Calcium hydroxide (0.5 equiv.) | T-5224 Form A | Calcium salt Form A | T-5224 Form A |
| Magnesium hydroxide (0.5 equiv.) | T-5224 Form A | T-5224 Form B | T-5224 Form A |
| L-Arginine (1.0 equiv.) | T-5224 Form A | L-Arginine salt, amorphous sample | L-Arginine salt, amorphous sample |
| Meglumine (1.0 equiv.) | Meglumine salt, amorphous sample | Turbid suspension | Meglumine salt, amorphous sample |
| tert-Butylamine (1.0 equiv.) | tert-Butylamine salt Form A | tert-Butylamine salt Form A | tert-Butylamine salt Form B |
| Ammonia (1.0 equiv.) | Ammonium salt Form A | Ammonium salt Form B | Ammonium salt Form A |
| Trometamol (1.0 equiv.) | T-5224 Form A | T-5224 Form A | T-5224 Form A |
| Choline (1.0 equiv.) | Choline salt Form A | Clear solution | T-5224 Form A |
| L-Lysine (1.0 equiv.) | T-5224 Form A | L-Lysine salt, amorphous sample | L-Lysine salt Form A |
| L-Lysine (1.0 equiv.) | // | // | L-Lysine salt Form A |
| Diethylamine (1.0 equiv.) | Diethylamine salt Form A | Clear solution | Diethylamine salt Form B |
| Dimethylethanolamine (1.0 equiv. or 2.0 equiv.) | Dimethylethanolamine salt Form A | Clear solution | T-5224 Form A |
| Betaine (1.0 equiv.) | Betaine salt Form A | Betaine salt Form A | T-5224 Form A |
| Ethylenediamine (1.0 equiv.) | Gel-like substance | Ethylenediamine salt Form A | Ethylenediamine salt Form B |

| | | | |
|---|---|---|---|
| Note: "//" indicates not performed. | | | |

### 2. Cooling Method

The turbid suspensions and clear solutions obtained from the suspension method above were cooled to 5°C to prepare solid samples for characterization. Only gel-like substances or clear solutions were obtained.

**Table 1-2: Cooling method**

| **Exp. No.** | **Base (equiv.)** | **Tetrahydrofuran** |
|---|---|---|
| C1 | Meglumine (1.0 equiv.) | Gel-like substance |
| C2 | Choline (1.0 equiv.) | Clear solution |
| C3 | Diethylamine (1.0 equiv.) | Clear solution |
| C4 | Dimethylethanolamine (1.0 equiv.) | Clear solution |

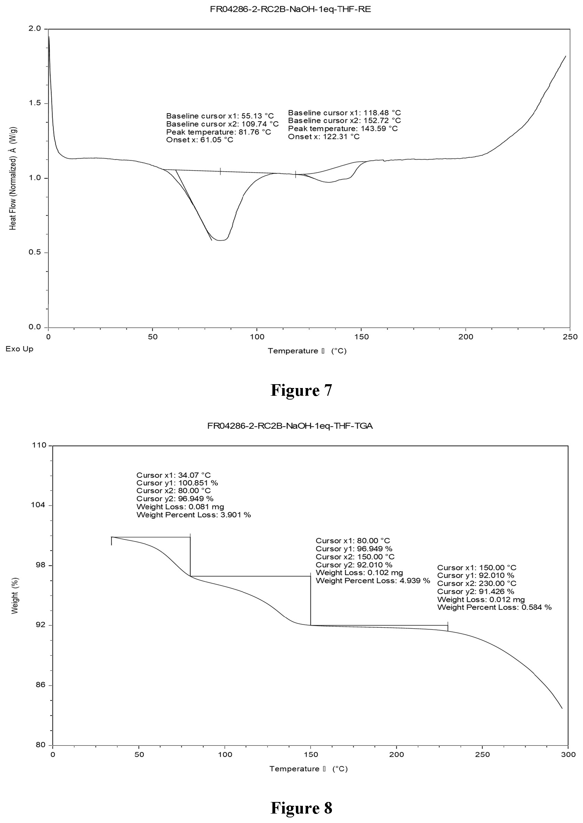

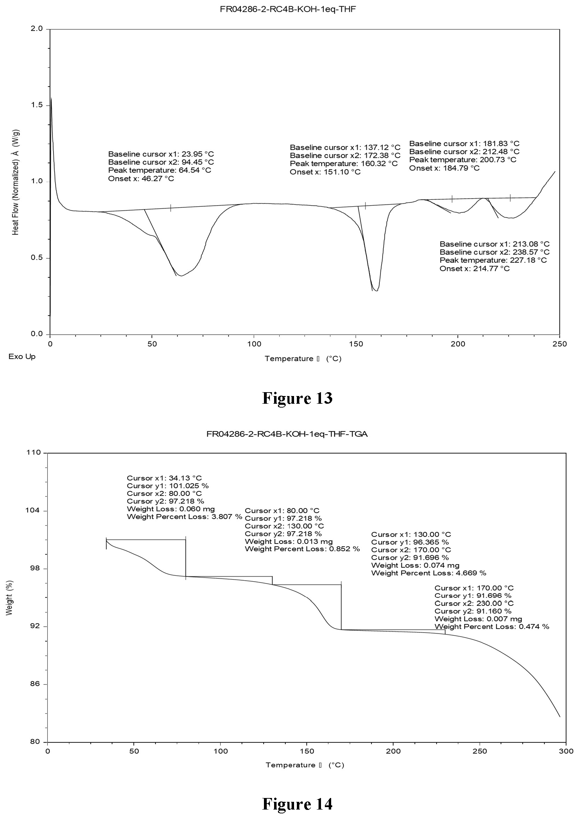

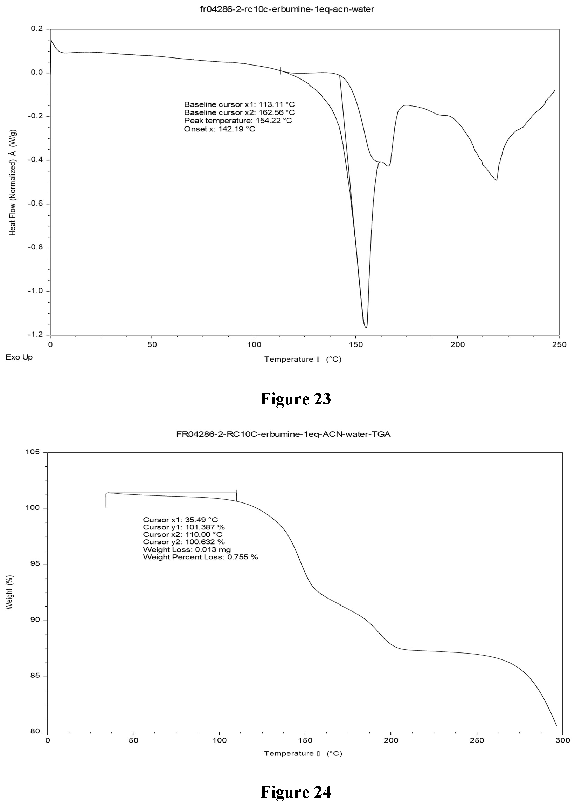

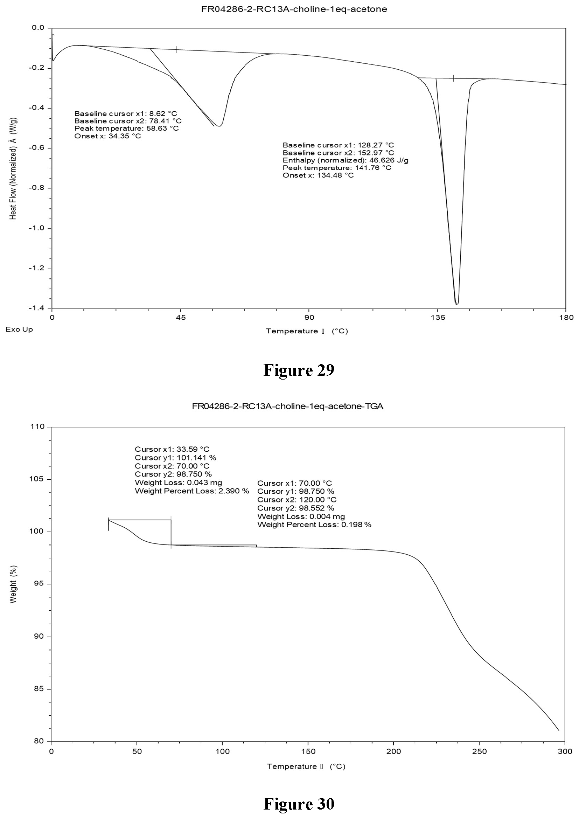

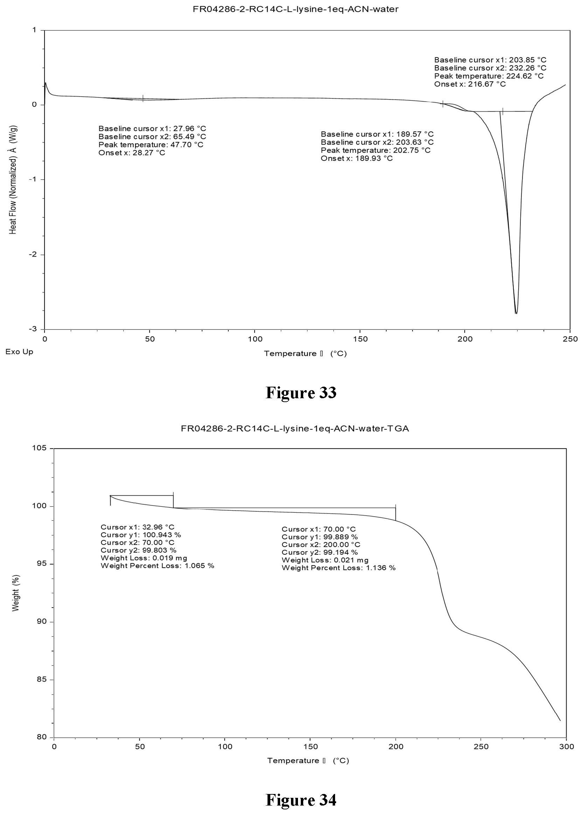

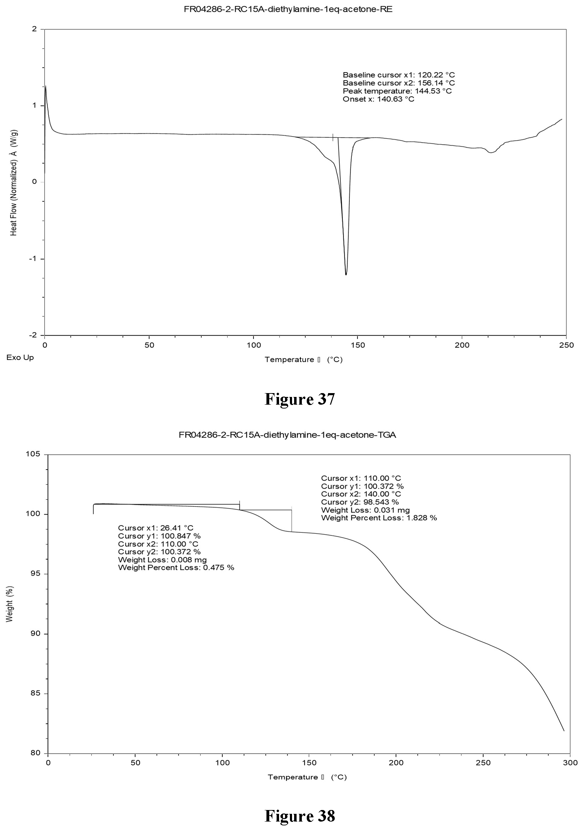

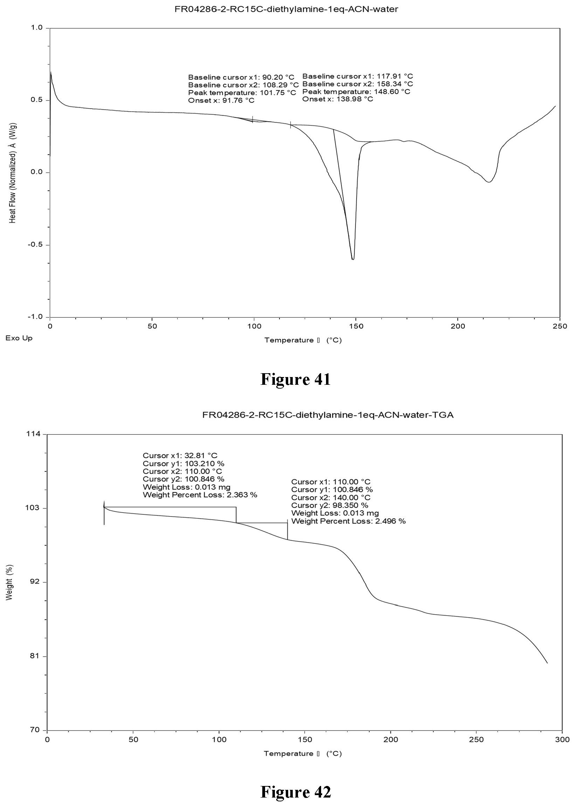

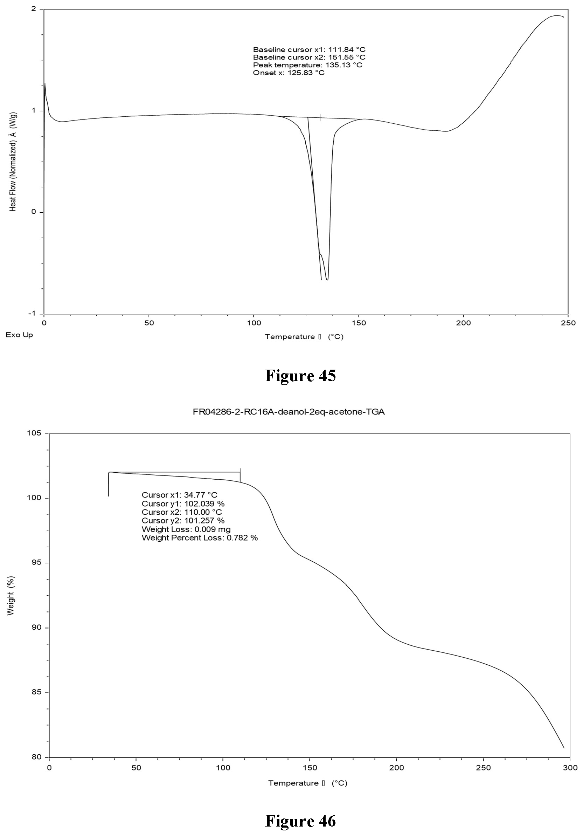

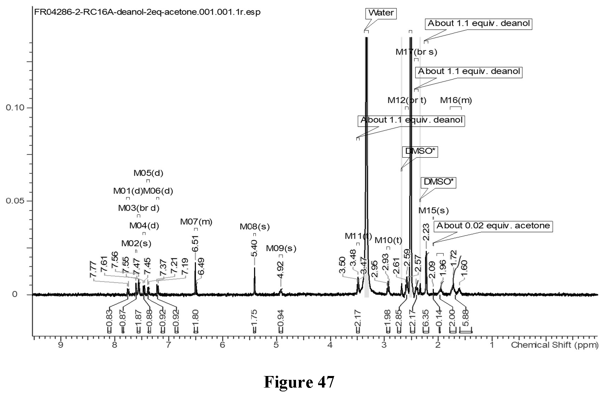

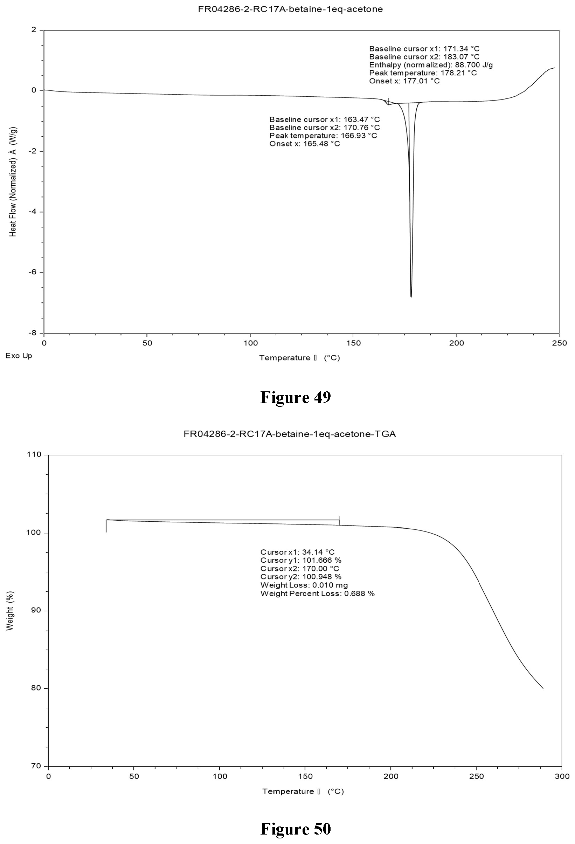

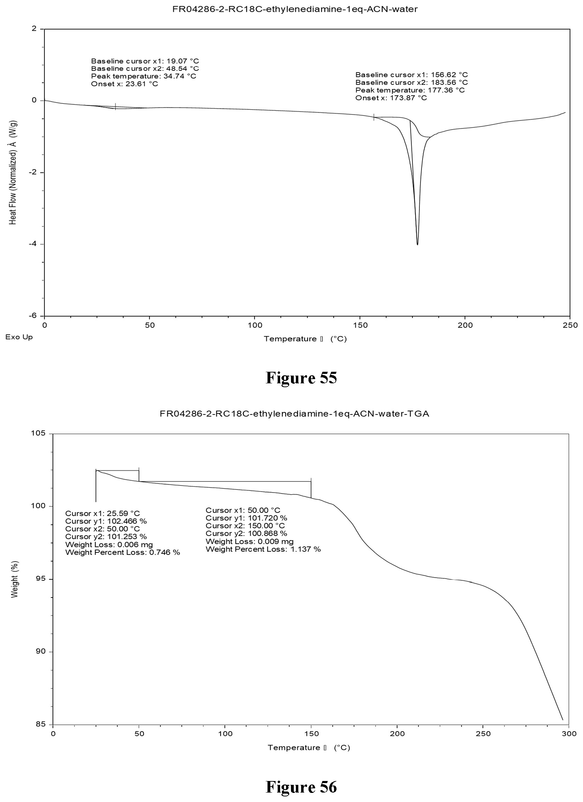

Based on the above screening experiment, sodium, potassium, calcium, tert-butylamine, ammonium, choline, L-lysine, diethylamine, dimethylethanolamine, betaine, and ethylenediamine salts of Compound T-5224, as well as their corresponding polymorphs, were prepared. Characterization of the crystal forms of the salts can be seen in Figures 4 to 57. Among them, the X-ray powder diffraction (XRPD) data for T-5224 betaine salt Form A and choline salt Form A are shown in Table A and Table B.

**Table A: X-ray powder diffraction data for T-5224 betaine salt Form A**

| **Index** | **Angle** | **d Value** | **Net Intensity** | **Gross Intensity** | **Rel. Intensity** |
|---|---|---|---|---|---|
| 1 | 3.984 ° | 22.16067 Å | 909.209 | 1213.02 | 4.6% |
| 2 | 7.885 ° | 11.20329 Å | 850.325 | 1254.54 | 4.3% |
| 3 | 8.140 ° | 10.85315 Å | 5092.07 | 5511.04 | 25.6% |
| 4 | 8.671 ° | 10.18938 Å | 17604.3 | 18046.7 | 88.6% |
| 5 | 10.712 ° | 8.25223 Å | 3373.36 | 3898.98 | 17.0% |
| 6 | 11.327 ° | 7.80568 Å | 185.089 | 745.222 | 0.9% |
| 7 | 11.799 ° | 7.49460 Å | 6709.13 | 7294.30 | 33.8% |
| 8 | 13.600 ° | 6.50586 Å | 735.369 | 1355.30 | 3.7% |
| 9 | 13.914 ° | 6.35936 Å | 50.7114 | 667.100 | 0.3% |
| 10 | 14.545 ° | 6.08508 Å | 2148.75 | 2812.19 | 10.8% |
| 11 | 14.981 ° | 5.90894 Å | 1374.74 | 2092.85 | 6.9% |
| 12 | 15.442 ° | 5.73366 Å | 8093.70 | 8862.38 | 40.7% |
| 13 | 15.666 ° | 5.65209 Å | 2450.57 | 3241.20 | 12.3% |
| 14 | 15.890 ° | 5.57281 Å | 2209.16 | 3019.99 | 11.1% |
| 15 | 16.311 ° | 5.42998 Å | 19862.1 | 20706.1 | 100.0% |
| 16 | 16.983 ° | 5.21666 Å | 1028.29 | 1912.51 | 5.2% |
| 17 | 17.159 ° | 5.16361 Å | 1660.25 | 2552.41 | 8.4% |
| 18 | 17.326 ° | 5.11413 Å | 2265.37 | 3164.08 | 11.4% |
| 19 | 17.589 ° | 5.03835 Å | 3413.40 | 4320.43 | 17.2% |
| 20 | 17.870 ° | 4.95971 Å | 2238.64 | 3151.91 | 11.3% |
| 21 | 18.381 ° | 4.82276 Å | 6285.29 | 7213.93 | 31.6% |
| 22 | 18.766 ° | 4.72470 Å | 1509.03 | 2450.90 | 7.6% |
| 23 | 19.069 ° | 4.65040 Å | 4268.48 | 5217.15 | 21.5% |
| 24 | 19.608 ° | 4.52377 Å | 8482.02 | 9434.90 | 42.7% |
| 25 | 19.627 ° | 4.51952 Å | 7360.75 | 8313.61 | 37.1% |
| 26 | 20.431 ° | 4.34328 Å | 7126.17 | 8096.08 | 35.9% |
| 27 | 21.045 ° | 4.21801 Å | 3082.93 | 4074.80 | 15.5% |
| 28 | 21.440 ° | 4.14124 Å | 14221.6 | 15220.7 | 71.6% |
| 29 | 21.707 ° | 4.09094 Å | 3286.41 | 4287.28 | 16.5% |
| 30 | 22.043 ° | 4.02918 Å | 4597.74 | 5597.35 | 23.1% |
| 31 | 22.331 ° | 3.97785 Å | 7779.57 | 8774.97 | 39.2% |
| 32 | 22.684 ° | 3.91678 Å | 4869.03 | 5855.34 | 24.5% |
| 33 | 23.016 ° | 3.86114 Å | 180.997 | 1154.84 | 0.9% |
| 34 | 23.389 ° | 3.80034 Å | 10352.5 | 11307.7 | 52.1% |
| 35 | 23.619 ° | 3.76384 Å | 1740.23 | 2681.53 | 8.8% |
| 36 | 24.019 ° | 3.70206 Å | 4661.61 | 5574.39 | 23.5% |
| 37 | 24.233 ° | 3.66979 Å | 4939.25 | 5834.43 | 24.9% |
| 38 | 24.784 ° | 3.58951 Å | 3044.74 | 3887.44 | 15.3% |
| 39 | 25.857 ° | 3.44297 Å | 5547.58 | 6342.78 | 27.9% |
| 40 | 26.258 ° | 3.39126 Å | 418.575 | 1208.69 | 2.1% |
| 41 | 26.715 ° | 3.33421 Å | 3612.03 | 4389.52 | 18.2% |
| 42 | 27.254 ° | 3.26947 Å | 1549.25 | 2302.52 | 7.8% |
| 43 | 27.666 ° | 3.22175 Å | 2764.83 | 3492.81 | 13.9% |
| 44 | 27.966 ° | 3.18790 Å | 704.871 | 1410.70 | 3.5% |
| 45 | 29.267 ° | 3.04909 Å | 1284.14 | 1956.52 | 6.5% |
| 46 | 29.714 ° | 3.00424 Å | 3502.57 | 4179.07 | 17.6% |
| 47 | 30.067 ° | 2.96975 Å | 3458.62 | 4133.46 | 17.4% |
| 48 | 30.660 ° | 2.91363 Å | 2502.41 | 3164.71 | 12.6% |
| 49 | 31.032 ° | 2.87954 Å | 179.984 | 828.166 | 0.9% |
| 50 | 31.600 ° | 2.82904 Å | 351.459 | 996.177 | 1.8% |
| 51 | 31.877 ° | 2.80508 Å | 783.960 | 1437.46 | 3.9% |
| 52 | 32.367 ° | 2.76373 Å | 667.534 | 1330.04 | 3.4% |
| 53 | 32.554 ° | 2.74831 Å | 748.584 | 1412.33 | 3.8% |
| 54 | 32.872 ° | 2.72248 Å | 946.203 | 1609.26 | 4.8% |
| 55 | 33.346 ° | 2.68484 Å | 413.059 | 1068.58 | 2.1% |
| 56 | 33.651 ° | 2.66122 Å | 691.500 | 1338.04 | 3.5% |
| 57 | 34.122 ° | 2.62555 Å | 1813.28 | 2439.59 | 9.1% |
| 58 | 34.543 ° | 2.59447 Å | 613.157 | 1214.82 | 3.1% |
| 59 | 35.146 ° | 2.55135 Å | 345.569 | 912.502 | 1.7% |
| 60 | 35.790 ° | 2.50686 Å | 566.471 | 1111.23 | 2.9% |
| 61 | 36.188 ° | 2.48021 Å | 83.9513 | 607.812 | 0.4% |
| 62 | 36.532 ° | 2.45768 Å | 80.0010 | 595.361 | 0.4% |
| 63 | 37.242 ° | 2.41243 Å | 569.908 | 1088.77 | 2.9% |
| 64 | 37.879 ° | 2.37327 Å | 244.704 | 757.628 | 1.2% |
| 65 | 37.985 ° | 2.36694 Å | 357.274 | 867.868 | 1.8% |
| 66 | 38.315 ° | 2.34728 Å | 162.670 | 663.415 | 0.8% |
| 67 | 39.375 ° | 2.28648 Å | 393.305 | 906.016 | 2.0% |
| 68 | 39.650 ° | 2.27126 Å | 263.538 | 803.070 | 1.3% |

**Table B: X-ray powder diffraction data for T-5224 choline salt Form A**

| **Index** | **Angle** | **d Value** | **Net Intensity** | **Gross Intensity** | **Rel. Intensity** |
|---|---|---|---|---|---|
| 1 | 3.456 ° | 25.54531 Å | 910.584 | 1156.41 | 8.0% |
| 2 | 6.870 ° | 12.85587 Å | 5284.00 | 5498.95 | 46.5% |
| 3 | 9.357 ° | 9.44448 Å | 70.3943 | 321.364 | 0.6% |
| 4 | 10.010 ° | 8.82947 Å | 922.222 | 1194.71 | 8.1% |
| 5 | 10.299 ° | 8.58222 Å | 607.834 | 886.355 | 5.3% |
| 6 | 11.677 ° | 7.57235 Å | 2630.55 | 2936.14 | 23.1% |
| 7 | 13.726 ° | 6.44620 Å | 525.467 | 860.448 | 4.6% |
| 8 | 14.008 ° | 6.31701 Å | 5807.66 | 6149.24 | 51.1% |
| 9 | 14.966 ° | 5.91469 Å | 2512.83 | 2861.63 | 22.1% |
| 10 | 16.550 ° | 5.35210 Å | 907.434 | 1328.25 | 8.0% |
| 11 | 16.737 ° | 5.29268 Å | 1841.06 | 2276.86 | 16.2% |
| 12 | 17.177 ° | 5.15805 Å | 1987.89 | 2455.40 | 17.5% |
| 13 | 17.670 ° | 5.01531 Å | 3315.57 | 3812.68 | 29.2% |
| 14 | 18.572 ° | 4.77382 Å | 3166.52 | 3711.49 | 27.9% |
| 15 | 19.140 ° | 4.63336 Å | 3278.92 | 3853.80 | 28.9% |
| 16 | 19.700 ° | 4.50294 Å | 4973.21 | 5569.47 | 43.8% |
| 17 | 19.837 ° | 4.47197 Å | 530.091 | 1130.39 | 4.7% |
| 18 | 20.898 ° | 4.24738 Å | 2213.82 | 2856.52 | 19.5% |
| 19 | 21.246 ° | 4.17849 Å | 5046.01 | 5707.56 | 44.4% |
| 20 | 21.756 ° | 4.08177 Å | 878.191 | 1561.69 | 7.7% |
| 21 | 22.794 ° | 3.89823 Å | 11363.7 | 12071.4 | 100.0% |
| 22 | 23.244 ° | 3.82370 Å | 330.245 | 1039.76 | 2.9% |
| 23 | 23.803 ° | 3.73510 Å | 1067.69 | 1772.32 | 9.4% |
| 24 | 24.115 ° | 3.68759 Å | 1561.00 | 2259.45 | 13.7% |
| 25 | 24.720 ° | 3.59869 Å | 542.882 | 1231.44 | 4.8% |
| 26 | 25.154 ° | 3.53746 Å | 1550.38 | 2233.10 | 13.6% |
| 27 | 25.339 ° | 3.51211 Å | 659.539 | 1338.31 | 5.8% |
| 28 | 26.050 ° | 3.41784 Å | 922.268 | 1605.91 | 8.1% |
| 29 | 26.278 ° | 3.38867 Å | 2195.40 | 2882.81 | 19.3% |
| 30 | 26.472 ° | 3.36433 Å | 3414.59 | 4104.14 | 30.0% |
| 31 | 27.020 ° | 3.29730 Å | 4163.58 | 4854.00 | 36.6% |
| 32 | 27.615 ° | 3.22758 Å | 1937.89 | 2620.54 | 17.1% |
| 33 | 28.258 ° | 3.15562 Å | 357.740 | 1021.80 | 3.1% |
| 34 | 28.507 ° | 3.12859 Å | 506.037 | 1160.04 | 4.5% |
| 35 | 29.348 ° | 3.04082 Å | 560.725 | 1191.58 | 4.9% |
| 36 | 29.542 ° | 3.02132 Å | 1033.08 | 1660.56 | 9.1% |
| 37 | 30.290 ° | 2.94834 Å | 174.295 | 779.682 | 1.5% |
| 38 | 30.725 ° | 2.90757 Å | 128.675 | 718.723 | 1.1% |
| 39 | 31.400 ° | 2.84661 Å | 76.9995 | 642.144 | 0.7% |
| 40 | 31.604 ° | 2.82872 Å | 76.3895 | 632.212 | 0.7% |
| 41 | 32.109 ° | 2.78540 Å | 573.178 | 1124.04 | 5.0% |
| 42 | 33.351 ° | 2.68446 Å | 526.877 | 1067.29 | 4.6% |
| 43 | 34.142 ° | 2.62400 Å | 89.6433 | 602.755 | 0.8% |
| 44 | 34.355 ° | 2.60824 Å | 168.996 | 672.044 | 1.5% |
| 45 | 35.325 ° | 2.53883 Å | 120.791 | 587.573 | 1.1% |
| 46 | 35.759 ° | 2.50902 Å | 206.479 | 671.386 | 1.8% |
| 47 | 36.152 ° | 2.48261 Å | 343.448 | 802.493 | 3.0% |
| 48 | 36.407 ° | 2.46581 Å | 142.926 | 596.053 | 1.3% |
| 49 | 37.115 ° | 2.42037 Å | 133.798 | 598.945 | 1.2% |
| 50 | 37.380 ° | 2.40385 Å | 171.816 | 645.873 | 1.5% |
| 51 | 37.928 ° | 2.37032 Å | 205.611 | 698.556 | 1.8% |
| 52 | 38.634 ° | 2.32864 Å | 444.763 | 964.032 | 3.9% |
| 53 | 39.309 ° | 2.29017 Å | 275.249 | 810.038 | 2.4% |
| 54 | 39.638 ° | 2.27192 Å | 557.837 | 1095.95 | 4.9% |

### Example 2: Preliminary Study on Stability and Solubility of T-5224 Salts and Crystal Forms thereof

The sodium salt Form B, potassium salt Form B, tert-butylamine salt Form B, choline salt Form A, L-lysine salt Form A, diethylamine salt Form A, diethylamine salt Form B, dimethylethanolamine salt Form A, betaine salt Form A, and ethylenediamine salt Form B prepared in Example 1 exhibited good crystallinity and reasonable stoichiometry. Therefore, the polymorphs of these salts were selected for stability studies.

The candidate salt forms were exposed at 25°C/60% RH for 3 days and at 25°C/80% RH for 2 days to preliminarily investigate the physical stability of these 10 candidate salt forms. Additionally, approximate solubility experiments in pH 4.5 acetate buffer solution were conducted for these 10 candidate salt forms. Candidate salt forms were selected for further evaluation based on physical stability and solubility. The results are shown in Tables 2-1 and 2-2.

**Table 2-1**

| | Sodium salt Form B | Potassium salt Form B | tert-Butylamine salt Form B | Choline salt Form A |
|---|---|---|---|---|
| **Solvent** | Tetrahydrofuran | Tetrahydrofuran | Acetonitrile/Water (95:5, v:v) | Acetone |
| **Crystallinity (XRPD)** | High crystallinity | High crystallinity | High crystallinity | High crystallinity |
| **Results** | 25°C/60%RH, open storage for 3 days: | 25°C/60%RH, open storage for 3 days: | 25°C/60%RH, open storage for 3 days: | 25°C/60%RH, open storage for 3 days: |
| | No significant moisture absorption of the solid; no significant color change. | No significant moisture absorption of the solid; no significant color change. | No significant moisture absorption of the solid; no significant color change. | No significant moisture absorption of the solid; no significant color change. |
| | 25°C/80%RH, open storage for 2 days: | 25°C/80%RH, open storage for 2 days: | 25°C/80%RH, open storage for 2 days: | 25°C/80%RH, open storage for 2 days: |
| | No significant moisture absorption of the solid; no significant color change. | No significant moisture absorption of the solid; no significant color change. | No significant moisture absorption of the solid; no significant color change. | No significant moisture absorption of the solid; no significant color change. |
| | Approximate solubility in pH 4.5 acetate buffer (50 mM): <0.25 mg/mL | Approximate solubility in pH 4.5 acetate buffer (50 mM): <0.25 mg/mL | Approximate solubility in pH 4.5 acetate buffer (50 mM): <0.25 mg/mL | Approximate solubility in pH 4.5 acetate buffer (50 mM): <0.25 mg/mL; during the dissolution process, a transparent gel-like substance formed. |
| | Exact solubility: < LOQ | Exact solubility: < LOQ | | |

**Table 2-2**

| | L-Lysine salt Form A | Diethylamine salt Form A | Diethylamine salt Form B | Dimethyletha nolamine salt Form A | Betaine salt Form A | Ethylenediam ine salt Form B |
|---|---|---|---|---|---|---|
| **Solvent** | Acetonitrile/ Water (95:5, v:v) | Acetone | Acetonitrile/ Water (95:5, v:v) | Acetone | Acetone | Acetonitrile/ Water (95:5, v:v) |
| **Crystall inity (XRPD)** | Low crystallinity | High crystallinity | Medium crystallinity | High crystallinity | High crystallinity | High crystallinity |
| **Results** | 25°C/60%RH , open storage for 3 days: | 25°C/60%RH , open storage for 3 days: | 25°C/60%RH , open storage for 3 days: | 25°C/60%RH , open storage for 3 days: | 25°C/60%RH , open storage for 3 days: | - |
| | No significant moisture absorption of the solid; no significant color change. | No significant moisture absorption of the solid; no significant color change. | No significant moisture absorption of the solid; no significant color change. | No significant moisture absorption of the solid; no significant color change. | No significant moisture absorption of the solid; no significant color change. | |
| | 25°C/80%RH , open storage for 2 days: | 25°C/80%RH , open storage for 2 days: | 25°C/80%RH , open storage for 2 days: | 25°C/80%RH , open storage for 2 days: | 25°C/80%RH , open storage for 2 days: | |
| | No significant moisture absorption of the solid; no significant color change. | No significant moisture absorption of the solid; no significant color change. | No significant moisture absorption of the solid; no significant color change. | No significant moisture absorption of the solid; no significant color change. | No significant moisture absorption of the solid; no significant color change. | |
| | | Approximate solubility in pH 4.5 acetate buffer (50 mM): <0.25 mg/mL | | Approximate solubility in pH 4.5 acetate buffer (50 mM): <0.25 mg/mL | Approximate solubility in pH 4.5 acetate buffer (50 mM): <0.25 mg/mL | |

Among the candidate salt forms listed above, the choline salt Form A, diethylamine salt Form A, and betaine salt Form A exhibited good physicochemical properties including good crystallinity and good physical stability. Furthermore, in the approximate solubility experiment in pH 4.5 acetate buffer solution at 25°C, they also showed potential advantages in solubility. Therefore, these three candidate salt forms were selected for scale-up preparation, and their stability, solubility, and hygroscopicity were systematically evaluated and compared with those of Compound T-5224 Form A.

### Example 3: Comparison of Physicochemical Properties of Advantageous Salt Forms and Crystal Forms thereof with T-5224 Form A

### 1. Preparation of Control Compound and Candidate Salt Forms

In this section, scale-up batches of the choline salt Form A and betaine salt Form A were successfully obtained. The crystal forms of the scale-up candidate salt forms were consistent with those obtained in the preliminary screening. For the diethylamine salt, even with the addition of seeds of the diethylamine salt Form A, a new crystal form, diethylamine salt Form C, was obtained. This indicates that the diethylamine salt Form C is more stable than the diethylamine salt Form A. Therefore, subsequent systematic characterization was performed on the diethylamine salt Form C.

### 1.1 Preparation of control T-5224 Form A

500 mg of Compound T-5224 was weighed and placed into an 8 mL glass vial. 4 mL of acetone was added, and the mixture was stirred at 50°C for 5 minutes to obtain a suspension. (suspension)
Approximately 3 mg of T-5224 Form A seeds were added to the above suspension. (suspension)
The mixture was stirred at 50°C for 2 hours, then cooled to 25°C and stirred at 25°C for about 3 days. (suspension)

The solid part was collected by centrifugation, and the obtained solid was dried under vacuum at 50°C for about 2 hours.

Approximately 253 mg of Form A was obtained as an off-white powder with a yield of 51%.

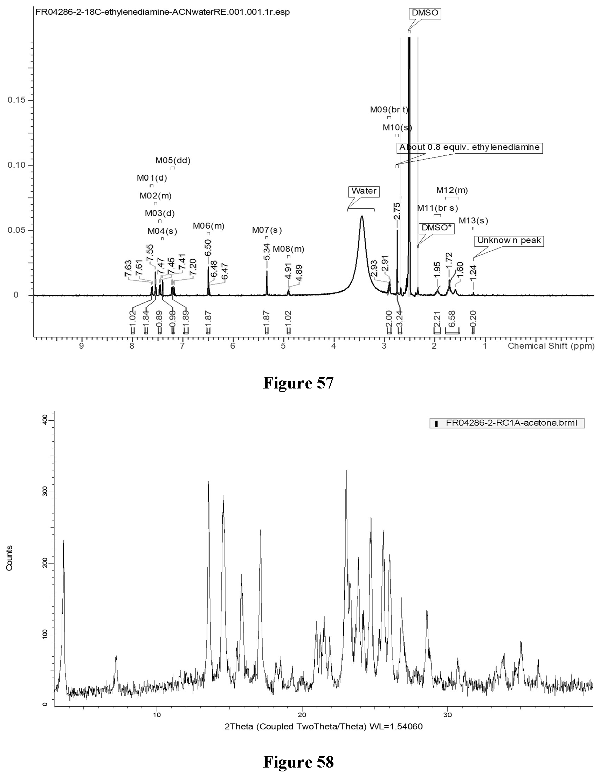

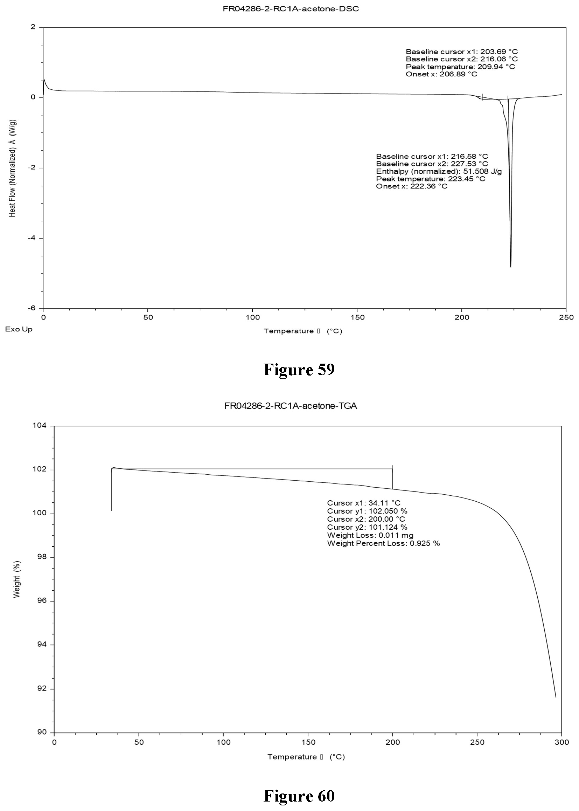

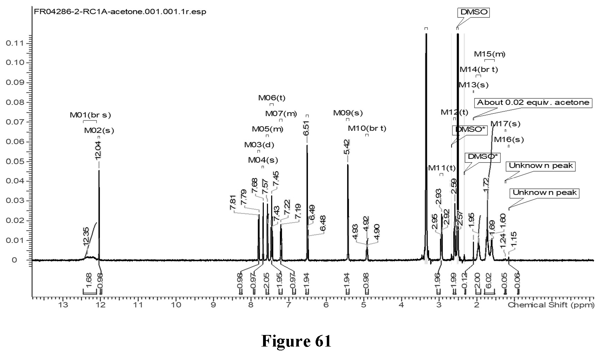

Characterization of the control T-5224 Form A is shown in Figures 58 to 61.

### 1.2 Preparation of T-5224 choline salt Form A

500 mg of Compound T-5224 was weighed and placed into an 8 mL glass vial with 256 mg (1.05 equiv.) of choline. 1 mL of acetone was added, and the mixture was stirred at 50°C for 5 minutes to obtain a clear solution. (clear solution)
After stirring at 50°C for 2 hours, the solution became turbid and gradually turned into a suspension. (clear solution → suspension)
Approximately 3 mg of choline salt Form A seeds were added to the above suspension. (suspension)
The suspension was cooled to 25°C and stirred at 25°C for about 2 days. (suspension)
The suspension was cooled to 5°C and stirred at 5°C for about 1 day. (suspension)

The solid part was collected by centrifugation, and the obtained solid was dried under vacuum at 50°C for about 2 hours.

Approximately 150 mg of choline salt Form A was obtained as an off-white powder with a yield of 20%.

### 1.3 Preparation of T-5224 diethylamine salt Form C

500 mg of Compound T-5224 was weighed and placed into an 8 mL glass vial with 71 mg (1.05 equiv.) of diethylamine. 5.1 mL of acetone was added, and the mixture was stirred at 50°C for 5 minutes to obtain a suspension. (suspension)
Approximately 3 mg of diethylamine salt Form A seeds were added to the above suspension. (suspension)
The suspension was stirred at 50°C for 2 hours, then cooled to 25°C and stirred at 25°C for about 2 days. (suspension)

The solid part was collected by centrifugation, and the obtained solid was dried under vacuum at 50°C for about 2 hours.

Approximately 523 mg of the new crystal form, diethylamine salt Form C, was obtained as an off-white powder with a yield of 92%.

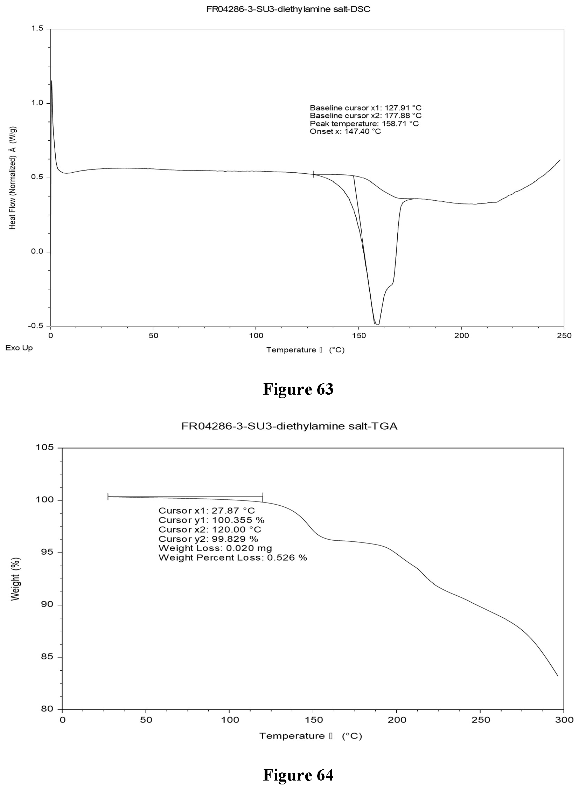

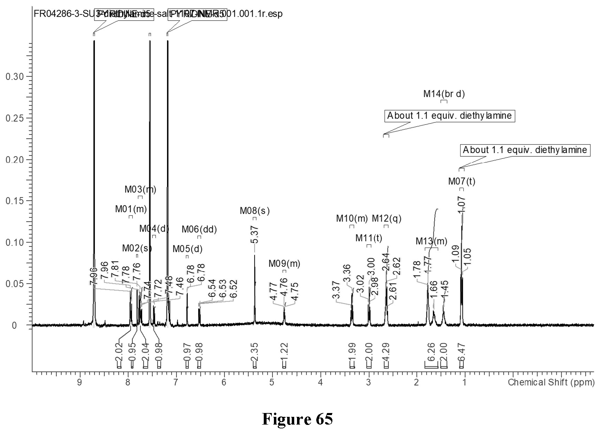

Characterization of T-5224 diethylamine salt Form C is shown in Figures 62 to 65. Among them, the X-ray powder diffraction (XRPD) data is shown in Table C.

**Table C: X-ray powder diffraction data for T-5224 diethylamine salt Form C**

| **Index** | **Angle** | **d Value** | **Net Intensity** | **Gross Intensity** | **Rel. Intensity** |
|---|---|---|---|---|---|
| 1 | 4.742 ° | 18.62135 Å | 7096.38 | 7563.28 | 44.1% |
| 2 | 6.424 ° | 13.74722 Å | 10903.8 | 11326.6 | 67.8% |
| 3 | 8.691 ° | 10.16568 Å | 145.074 | 624.172 | 0.9% |
| 4 | 9.121 ° | 9.68809 Å | 279.575 | 782.424 | 1.7% |
| 5 | 9.430 ° | 9.37136 Å | 11332.6 | 11849.1 | 70.4% |
| 6 | 9.885 ° | 8.94064 Å | 394.055 | 925.292 | 2.4% |
| 7 | 10.378 ° | 8.51677 Å | 450.973 | 991.092 | 2.8% |
| 8 | 12.066 ° | 7.32928 Å | 521.812 | 1131.90 | 3.2% |
| 9 | 12.808 ° | 6.90601 Å | 488.631 | 1145.64 | 3.0% |
| 10 | 13.126 ° | 6.73979 Å | 3099.73 | 3771.67 | 19.3% |
| 11 | 13.847 ° | 6.38998 Å | 6322.60 | 7017.07 | 39.3% |
| 12 | 14.143 ° | 6.25693 Å | 2410.74 | 3109.86 | 15.0% |
| 13 | 14.814 ° | 5.97507 Å | 1397.85 | 2097.64 | 8.7% |
| 14 | 15.018 ° | 5.89428 Å | 9882.41 | 10579.7 | 61.4% |
| 15 | 15.586 ° | 5.68085 Å | 68.5752 | 752.171 | 0.4% |
| 16 | 16.080 ° | 5.50757 Å | 1550.30 | 2249.37 | 9.6% |
| 17 | 16.611 ° | 5.33267 Å | 1294.81 | 2027.84 | 8.0% |
| 18 | 16.799 ° | 5.27326 Å | 6643.80 | 7386.82 | 41.3% |
| 19 | 17.331 ° | 5.11278 Å | 1416.49 | 2181.83 | 8.8% |
| 20 | 17.987 ° | 4.92772 Å | 3659.47 | 4451.05 | 22.7% |
| 21 | 18.945 ° | 4.68061 Å | 4142.82 | 4960.25 | 25.7% |
| 22 | 19.372 ° | 4.57837 Å | 2395.65 | 3215.60 | 14.9% |
| 23 | 19.747 ° | 4.49228 Å | 11423.2 | 12240.8 | 71.0% |
| 24 | 20.404 ° | 4.34903 Å | 16092.4 | 16895.4 | 100.0% |
| 25 | 20.725 ° | 4.28241 Å | 1735.37 | 2526.58 | 10.8% |
| 26 | 21.155 ° | 4.19641 Å | 609.016 | 1379.45 | 3.8% |
| 27 | 21.360 ° | 4.15645 Å | 262.088 | 1020.56 | 1.6% |
| 28 | 22.022 ° | 4.03297 Å | 547.554 | 1305.87 | 3.4% |
| 29 | 22.485 ° | 3.95107 Å | 2269.48 | 3055.05 | 14.1% |
| 30 | 22.966 ° | 3.86934 Å | 3635.40 | 4442.43 | 22.6% |
| 31 | 23.268 ° | 3.81977 Å | 3535.57 | 4352.47 | 22.0% |
| 32 | 23.657 ° | 3.75782 Å | 2019.17 | 2844.66 | 12.5% |
| 33 | 24.152 ° | 3.68197 Å | 1632.88 | 2462.65 | 10.1% |
| 34 | 24.506 ° | 3.62961 Å | 543.399 | 1371.66 | 3.4% |
| 35 | 24.869 ° | 3.57739 Å | 4339.25 | 5161.99 | 27.0% |
| 36 | 25.214 ° | 3.52929 Å | 903.404 | 1717.20 | 5.6% |
| 37 | 25.415 ° | 3.50175 Å | 681.665 | 1488.55 | 4.2% |
| 38 | 25.727 ° | 3.46006 Å | 157.978 | 951.748 | 1.0% |
| 39 | 25.994 ° | 3.42504 Å | 877.235 | 1657.38 | 5.5% |
| 40 | 26.148 ° | 3.40531 Å | 1343.08 | 2114.43 | 8.3% |
| 41 | 26.485 ° | 3.36263 Å | 1686.96 | 2436.41 | 10.5% |
| 42 | 26.705 ° | 3.33544 Å | 624.134 | 1357.47 | 3.9% |
| 43 | 27.216 ° | 3.27397 Å | 1082.19 | 1777.72 | 6.7% |
| 44 | 27.488 ° | 3.24225 Å | 993.353 | 1667.78 | 6.2% |
| 45 | 27.823 ° | 3.20396 Å | 269.759 | 915.062 | 1.7% |
| 46 | 28.087 ° | 3.17445 Å | 684.742 | 1304.69 | 4.3% |
| 47 | 28.640 ° | 3.11436 Å | 1898.32 | 2458.23 | 11.8% |
| 48 | 28.863 ° | 3.09084 Å | 1124.63 | 1657.75 | 7.0% |
| 49 | 29.797 ° | 2.99599 Å | 153.798 | 613.890 | 1.0% |
| 50 | 30.171 ° | 2.95972 Å | 504.905 | 969.264 | 3.1% |
| 51 | 30.521 ° | 2.92654 Å | 272.748 | 737.242 | 1.7% |
| 52 | 31.411 ° | 2.84564 Å | 689.218 | 1141.76 | 4.3% |
| 53 | 31.748 ° | 2.81624 Å | 168.374 | 614.086 | 1.0% |
| 54 | 32.092 ° | 2.78679 Å | 126.140 | 561.300 | 0.8% |
| 55 | 32.401 ° | 2.76097 Å | 244.300 | 666.946 | 1.5% |
| 56 | 32.657 ° | 2.73990 Å | 90.9494 | 500.995 | 0.6% |
| 57 | 33.567 ° | 2.66764 Å | 368.782 | 790.338 | 2.3% |
| 58 | 33.909 ° | 2.64153 Å | 485.318 | 912.610 | 3.0% |
| 59 | 34.817 ° | 2.57470 Å | 47.1724 | 485.579 | 0.3% |
| 60 | 35.065 ° | 2.55704 Å | 104.587 | 551.861 | 0.6% |
| 61 | 35.395 ° | 2.53397 Å | 164.685 | 620.847 | 1.0% |
| 62 | 35.979 ° | 2.49412 Å | 561.319 | 1025.10 | 3.5% |
| 63 | 36.343 ° | 2.47001 Å | 89.4111 | 552.673 | 0.6% |
| 64 | 36.750 ° | 2.44357 Å | 224.016 | 687.263 | 1.4% |
| 65 | 37.257 ° | 2.41146 Å | 313.917 | 777.999 | 2.0% |
| 66 | 37.747 ° | 2.38129 Å | 153.223 | 610.674 | 1.0% |
| 67 | 38.187 ° | 2.35486 Å | 398.752 | 844.014 | 2.5% |
| 68 | 38.454 ° | 2.33914 Å | 256.059 | 691.075 | 1.6% |
| 69 | 38.977 ° | 2.30892 Å | 216.155 | 637.579 | 1.3% |
| 70 | 39.712 ° | 2.26786 Å | 178.151 | 683.156 | 1.1% |

### 1.4 Preparation of T-5224 betaine salt Form A

500 mg of Compound T-5224 was weighed and placed into an 8 mL glass vial with 122 mg (1.05 equiv.) of betaine. 4.4 mL of acetone was added, and the mixture was stirred at 50°C for 5 minutes to obtain a suspension. (suspension)
Approximately 3 mg of betaine salt Form A seeds were added to the above suspension. (suspension)
The suspension was stirred at 50°C for 2 hours, then cooled to 25°C and stirred at 25°C for about 2 days. (suspension)

The solid part was collected by centrifugation, and the obtained solid was dried under vacuum at 50°C for about 2 hours.

Approximately 473 mg of betaine salt Form A was obtained as an off-white powder with a yield of 76%.

### 2. Stability Comparison

### 2.1 Initial Chemical Purity

The chemical purity of T-5224 Form A, choline salt Form A, diethylamine salt Form C, and betaine salt Form A was 98.7%, 99.4%, 98.4%, and 98.8%, respectively.

### 2.2 Solid Stability

Open containers containing T-5224 Form A and the three candidate salt forms were placed at 25°C/92.5%RH for 1 week. Closed containers containing T-5224 Form A and the three candidate salt forms were placed at 60°C for 1 week. After the solid stability assessment, the samples were characterized by XRPD and HPLC, and changes in sample color were observed.

**Table 3-1: Solid stability, purity and appearance (color, CL)**

| | Solid Form | | | | | | | |
|---|---|---|---|---|---|---|---|---|
| | T-5224 Form A | | Choline salt Form A | | Diethylamine salt Form C | | Betaine salt Form A | |
| Initial Purity (HPLC) | 98.7% | | 99.4% | | 98.4% | | 98.8% | |
| Initial Color | Off-white | | Off-white | | Off-white | | Off-white | |
| | Purity | CL | Purity | CL | Purity | CL | Purity | CL |
| Solid, 25°C/92.5%RH, Open storage conditions, 1 week | | | | | | | | |
| | T-5224 Form A | | Choline salt Form A | | Diethylamine salt Form C | | Betaine salt Form A | |
| Initial Purity (HPLC) | 98.7% | | 99.4% | | 98.4% | | 98.8% | |
| Initial Color | Off-white | | Off-white | | Off-white | | Off-white | |
| | Purity | CL | Purity | CL | Purity | CL | Purity | CL |
| Solid (HPLC) | 98.7% | A | 99.5% | A | 98.4% | A | 98.7% | A |
| Solid (XRPD) | T-5224 Form A | | Choline salt Form A | | Diethylamine salt Form C | | Betaine salt Form A | |

| Solid, 60°C, closed container, 1 week | | | | | | | | |
|---|---|---|---|---|---|---|---|---|
| Solid (HPLC) | 98.7% | A | 99.5% | A | 98.4% | A | 98.7% | A |
| Solid (XRPD) | T-5224 Form A | | Choline salt Form A | | Diethylamine salt Form C | | Betaine salt Form A | |

| | | | | | | | | |
|---|---|---|---|---|---|---|---|---|
| Note: A: No color change; B: Slight color change; C: Moderate color change; D: Severe color change | | | | | | | | |

All the above physical forms exhibited good physical and chemical stability.

### 2.3 Solubility

In this section, the solubility of T-5224 Form A and the three candidate salt forms in water, five aqueous buffers, and simulated biological fluids at two time points (2 h and 24 h) at 37°C were studied. The selected media included pH 4.5 acetate buffer (50 mM) (only used for testing the solubility at 37°C for 2 h), water, pH 1.6 FaSSGF, pH 6.5 FaSSIF-v1, and pH 5.0 FeSSIF-v1. The residual solids from the solubility experiments were characterized by XRPD.

10.0 mg of T-5224 Form A, 12.6 mg of choline salt Form A, 11.3 mg of diethylamine salt Form C, or 12.3 mg of betaine salt Form A was precisely weighed and placed into 8 mL glass vials. 5 mL of the respective medium was added. The mass of salt weighed corresponded to 10 mg of T-5224 Form A. The resulting suspensions were stirred at 37°C at 400 rpm, and samples were taken at 2 hours and 24 hours. The samples were centrifuged at 14,000 rpm for 5 minutes at 37°C. The supernatant concentration was determined by HPLC, and the pH of the supernatant was measured using a pH meter. The results are shown in Table 3-2.

**Table 3-2: Solubility**

| Solubility at 37°C; Target concentration: 2mg/mL (as T-5224); Equilibration for 24 hours; LOQ: 0.5 µg/mL | | | | | | | | | | | |
|---|---|---|---|---|---|---|---|---|---|---|---|
| | Physical form | | | | | | | | | | |
| | T-5224 Form A | | Choline salt Form A | | Diethylamine salt Form C | | | Betaine salt Form A | | | |
| Solution | Solubility | | Solubility | | Solubility | | | Solubility | | | |
| medium | 2h | 24h (pH) | | 2h | 24h (pH) | | 2h | 24h (pH) | | 2h | 24h (pH) |
| Water | 1.4µg/m L | 1.4µg/m L (7.1) | | 0.7mg/ mL | 0.8mg/ mL (8.2) | | 0.8mg/ mL | 0.7mg/ mL (8.1) | | 6.3µg/m L | 4.3µg/m L (7.4) |
| FaSSGF, pH 1.6 | <LOQ | <LOQ (1.4) | | <LOQ | <LOQ (1.5) | | <LOQ | <LOQ (1.5) | | <LOQ | <LOQ (1.5) |
| FaSSIF-v1, pH 6.5 | 1.9µg/m L | 6.8µg/m L (6.5) | | 34.6µg/ mL | 26.3µg/ mL (6.6) | | 49.1µg/ mL | 22.7µg/ mL (6.5) | | 25.2µg/ mL | 13.2µg/ mL (6.3) |
| FeSSIF-v1, pH 5.0 | 16.2µg/ mL | 17.9µg/ mL (5.0) | | 15.8µg/ mL | 20.0µg/ mL (5.1) | | 15.1µg/ mL | 15.4µg/ mL (5.0) | | 15.2µg/ mL | 14.2µg/ mL (4.9) |
| pH 4.5 Acetate buffer (50mM) | 2.8µg/m L | // | | 2.4µg/m L | // | | 2.6µg/m L | // | | <LOQ | // |

| | | | | | | | | | | | |
|---|---|---|---|---|---|---|---|---|---|---|---|
| Note: "//": not performed. | | | | | | | | | | | |

The solubility of T-5224 Form A and the three candidate salt forms was pH-dependent. The three candidate salt forms showed comparable solubility to T-5224 in pH 1.6 FaSSGF, pH 4.5 acetate buffer (50 mM), and pH 5.0 FeSSIF-v1. The solubility in pH 1.6 FaSSGF and pH 4.5 acetate buffer (50 mM) was near or below the detection limit, while the solubility in pH 5.0 FeSSIF-v1 was 15-20 µg/mL.

The solubility of the three candidate salt forms in pH 6.5 FaSSIF-v1 (15-35 µg/mL) was slightly higher than that of T-5224 Form A (approximately 7 µg/mL).

The solubility of choline salt Form A and diethylamine salt Form C in water (0.9-1 mg/mL) was much higher than that of T-5224 Form A and betaine salt Form A (1-5 µg/mL).

In summary, the three candidate salt forms exhibited good crystallinity, as well as good physical and chemical stability. Additionally, the three candidate salt forms showed significant advantages in solubility. However, the diethylamine salt had no advantage over the other two salt forms due to its poorer thermal stability (decomposition starting around 120°C). From a physicochemical properties perspective, choline salt and betaine salt were comparable, but considering the safety of the base, choline salt was selected as the preferred salt form for subsequent crystal form screening.

### Example 4: Screening of Choline Salt Crystal Forms and Comparison of Physicochemical Properties with T-5224 Form A

### 1. Preparation of Choline Salt Crystal Forms

### 1.1 Preparation of Choline Salt Crystal Form B

5.0 g of T-5224 Form A was weighed and placed into an 8 mL glass vial with approximately 2.6 g (1.05 equiv.) of choline. 5 mL of ethanol was added, and the mixture was stirred at 50°C for 5 minutes to obtain a suspension. (suspension)
Approximately 10 mg of choline salt Form B seeds were added to the above suspension. (suspension)
The suspension was cooled to 5°C and stirred at 5°C for about 3 days. (suspension)

A portion of the solid was taken for XRPD analysis, which indicated a physical mixture of choline salt Form A and free acid Form A. Approximately 0.3 g (0.1 equiv.) of choline was added, and the mixture was stirred for about 1 day.

A portion of the solid was taken for XRPD analysis, which indicated a physical mixture of choline salt Form B and free acid Form A.
The solvent was blown dry, and 13 mL of ethyl acetate was added. The mixture was stirred at 25°C for 5 minutes to obtain a suspension. (suspension)
Approximately 3 mg of choline salt Form B seeds were added to the above suspension. (suspension)
The suspension was stirred at 25°C for about 1 day. (suspension)

The solid part was collected by centrifugation, and the obtained solid was dried under vacuum at 50°C for about 2 hours.

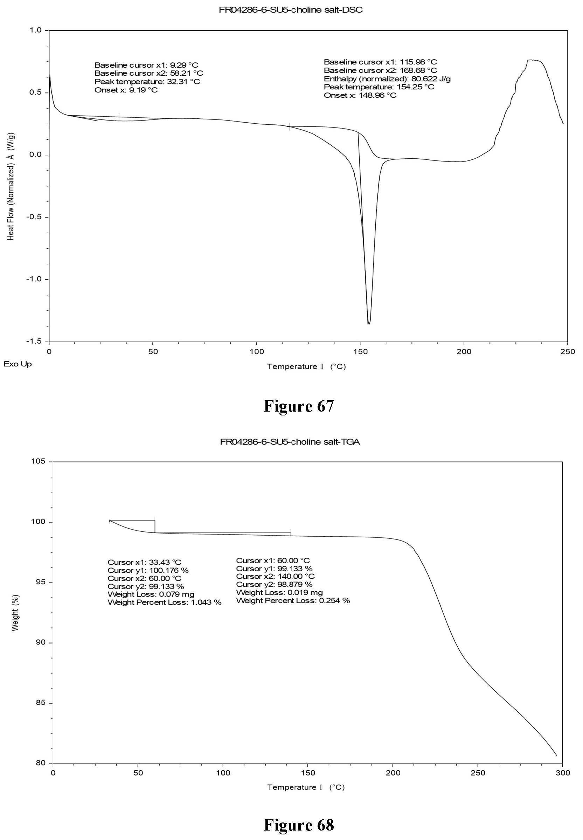

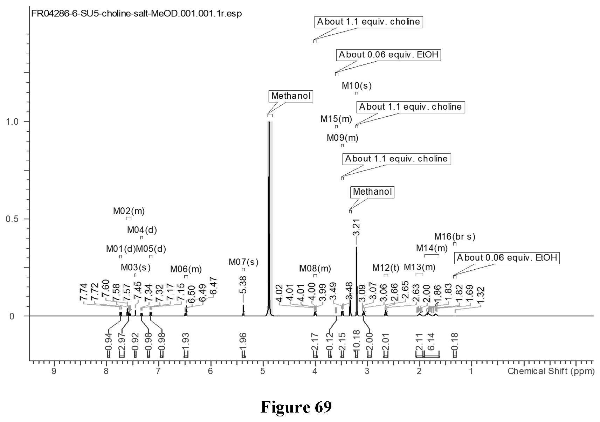

Approximately 5.2 g of choline salt Form B was obtained as an off-white powder with a yield of 66%. Characterization of choline salt Form B is shown in Figures 66 to 69. Among them, the X-ray powder diffraction (XRPD) data is shown in Table D.

**Table D: X-ray powder diffraction data for T-5224 choline salt Form B**

| **Index** | **Angle** | **d Value** | **Net Intensity** | **Gross Intensity** | **Rel. Intensity** |
|---|---|---|---|---|---|
| 1 | 3.541 ° | 24.93513 Å | 525.242 | 823.689 | 5.6% |
| 2 | 6.996 ° | 12.62499 Å | 651.721 | 940.612 | 6.9% |
| 3 | 8.556 ° | 10.32684 Å | 115.209 | 458.147 | 1.2% |
| 4 | 8.940 ° | 9.88324 Å | 5765.91 | 6119.58 | 61.3% |
| 5 | 10.053 ° | 8.79137 Å | 57.6623 | 444.422 | 0.6% |
| 6 | 10.458 ° | 8.45198 Å | 1981.21 | 2392.24 | 21.1% |
| 7 | 11.481 ° | 7.70134 Å | 1333.61 | 1795.29 | 14.2% |
| 8 | 12.177 ° | 7.26285 Å | 57.5570 | 551.768 | 0.6% |
| 9 | 12.643 ° | 6.99597 Å | 2193.44 | 2706.15 | 23.3% |
| 10 | 13.290 ° | 6.65664 Å | 1908.59 | 2438.33 | 20.3% |
| 11 | 13.936 ° | 6.34958 Å | 273.249 | 809.974 | 2.9% |
| 12 | 14.560 ° | 6.07871 Å | 286.266 | 840.673 | 3.0% |
| 13 | 15.493 ° | 5.71466 Å | 1813.92 | 2442.16 | 19.3% |
| 14 | 16.206 ° | 5.46482 Å | 3901.43 | 4614.96 | 41.5% |
| 15 | 16.462 ° | 5.38068 Å | 1147.44 | 1888.54 | 12.2% |
| 16 | 16.991 ° | 5.21424 Å | 4448.28 | 5241.57 | 47.3% |
| 17 | 17.374 ° | 5.09999 Å | 5007.91 | 5834.83 | 53.3% |
| 18 | 17.610 ° | 5.03216 Å | 1751.10 | 2596.97 | 18.6% |
| 19 | 18.034 ° | 4.91501 Å | 1389.45 | 2265.95 | 14.8% |
| 20 | 18.279 ° | 4.84945 Å | 1445.72 | 2338.02 | 15.4% |
| 21 | 18.887 ° | 4.69479 Å | 1555.21 | 2480.40 | 16.5% |
| 22 | 19.482 ° | 4.55268 Å | 8940.09 | 9888.91 | 95.1% |
| 23 | 19.913 ° | 4.45509 Å | 3598.62 | 4559.25 | 38.3% |
| 24 | 20.377 ° | 4.35467 Å | 9400.67 | 10369.0 | 100.0% |
| 25 | 20.924 ° | 4.24204 Å | 2792.66 | 3763.53 | 29.7% |
| 26 | 21.536 ° | 4.12285 Å | 1436.22 | 2401.38 | 15.3% |
| 27 | 21.796 ° | 4.07432 Å | 2274.54 | 3234.58 | 24.2% |
| 28 | 22.195 ° | 4.00194 Å | 4996.92 | 5945.91 | 53.2% |
| 29 | 22.512 ° | 3.94627 Å | 2221.79 | 3159.28 | 23.6% |
| 30 | 22.720 ° | 3.91072 Å | 7518.57 | 8447.24 | 80.0% |
| 31 | 23.030 ° | 3.85869 Å | 664.530 | 1578.07 | 7.1% |
| 32 | 23.325 ° | 3.81053 Å | 1042.58 | 1939.60 | 11.1% |
| 33 | 23.687 ° | 3.75317 Å | 104.173 | 978.095 | 1.1% |
| 34 | 23.996 ° | 3.70552 Å | 379.404 | 1253.85 | 4.0% |
| 35 | 24.405 ° | 3.64435 Å | 1851.88 | 2741.31 | 19.7% |
| 36 | 24.947 ° | 3.56641 Å | 1282.39 | 2185.51 | 13.6% |
| 37 | 25.542 ° | 3.48461 Å | 1677.74 | 2587.77 | 17.8% |
| 38 | 25.668 ° | 3.46788 Å | 1577.95 | 2488.35 | 16.8% |
| 39 | 26.045 ° | 3.41845 Å | 1338.11 | 2247.37 | 14.2% |
| 40 | 26.536 ° | 3.35637 Å | 2197.95 | 3100.63 | 23.4% |
| 41 | 26.886 ° | 3.31342 Å | 1180.32 | 2074.75 | 12.6% |
| 42 | 27.165 ° | 3.28005 Å | 6110.00 | 6995.77 | 65.0% |
| 43 | 27.533 ° | 3.23701 Å | 2718.66 | 3590.14 | 28.9% |
| 44 | 28.098 ° | 3.17322 Å | 896.174 | 1739.42 | 9.5% |
| 45 | 28.443 ° | 3.13548 Å | 1663.61 | 2485.85 | 17.7% |
| 46 | 29.278 ° | 3.04791 Å | 1408.18 | 2167.73 | 15.0% |
| 47 | 29.893 ° | 2.98665 Å | 664.393 | 1391.50 | 7.1% |
| 48 | 30.326 ° | 2.94492 Å | 744.162 | 1463.09 | 7.9% |
| 49 | 30.712 ° | 2.90881 Å | 98.7875 | 806.665 | 1.1% |
| 50 | 31.076 ° | 2.87558 Å | 267.479 | 966.370 | 2.8% |
| 51 | 31.515 ° | 2.83652 Å | 761.296 | 1449.91 | 8.1% |
| 52 | 31.680 ° | 2.82211 Å | 786.566 | 1470.12 | 8.4% |
| 53 | 31.984 ° | 2.79599 Å | 636.296 | 1308.84 | 6.8% |
| 54 | 32.350 ° | 2.76519 Å | 234.763 | 891.112 | 2.5% |
| 55 | 32.599 ° | 2.74465 Å | 171.216 | 814.703 | 1.8% |
| 56 | 33.297 ° | 2.68865 Å | 354.734 | 979.245 | 3.8% |
| 57 | 33.506 ° | 2.67239 Å | 203.140 | 823.706 | 2.2% |
| 58 | 34.135 ° | 2.62453 Å | 385.429 | 1011.50 | 4.1% |
| 59 | 34.325 ° | 2.61049 Å | 418.664 | 1049.43 | 4.5% |
| 60 | 34.769 ° | 2.57815 Å | 141.527 | 779.953 | 1.5% |
| 61 | 35.096 ° | 2.55488 Å | 113.977 | 755.019 | 1.2% |
| 62 | 35.544 ° | 2.52369 Å | 185.365 | 825.831 | 2.0% |
| 63 | 36.071 ° | 2.48798 Å | 652.653 | 1295.14 | 6.9% |
| 64 | 36.433 ° | 2.46413 Å | 160.515 | 812.061 | 1.7% |
| 65 | 37.252 ° | 2.41180 Å | 276.524 | 936.696 | 2.9% |
| 66 | 37.685 ° | 2.38508 Å | 299.224 | 957.417 | 3.2% |
| 67 | 38.283 ° | 2.34918 Å | 95.4094 | 744.977 | 1.0% |
| 68 | 38.541 ° | 2.33404 Å | 291.997 | 936.460 | 3.1% |
| 69 | 38.831 ° | 2.31728 Å | 221.849 | 858.662 | 2.4% |
| 70 | 39.405 ° | 2.28482 Å | 142.347 | 758.083 | 1.5% |

### 1.2 Preparation of Choline Salt Form A

See Example 1, Table 1-1.

### 1.3 Preparation of Choline Salt Form C

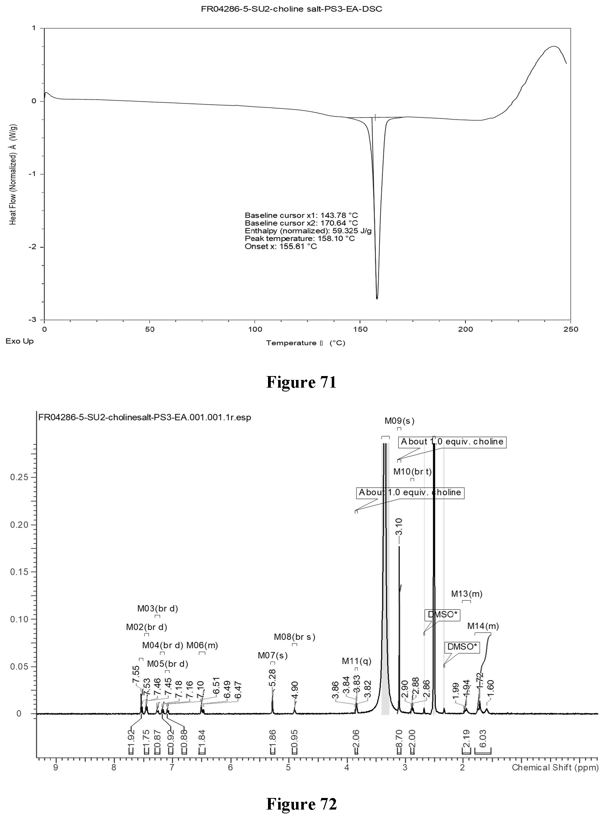

10 to 20 mg of choline salt Form A was weighed, and 100 to 300 µL of solvent ethyl acetate was added. The resulting suspension was suspended at 25°C for 2 days. The solid part was collected by centrifugation to obtain choline salt Form C. Characterization of choline salt Form C is shown in Figures 70 to 72. Among them, the X-ray powder diffraction (XRPD) data is shown in Table E.

**Table E: X-ray powder diffraction data for T-5224 choline salt Form C**

| **Index** | **Angle** | **d Value** | **Net Intensity** | **Gross Intensity** | **Rel. Intensity** |
|---|---|---|---|---|---|
| 1 | 4.703 ° | 18.77283 Å | 171.947 | 224.293 | 63.1% |
| 2 | 6.391 ° | 13.81980 Å | 219.095 | 271.971 | 80.4% |
| 3 | 9.401 ° | 9.40039 Å | 189.580 | 245.336 | 69.5% |
| 4 | 9.822 ° | 8.99836 Å | 18.1315 | 72.5436 | 6.7% |
| 5 | 10.280 ° | 8.59838 Å | 16.2760 | 69.5252 | 6.0% |
| 6 | 13.093 ° | 6.75654 Å | 73.1936 | 125.900 | 26.8% |
| 7 | 13.816 ° | 6.40448 Å | 85.5021 | 137.239 | 31.4% |
| 8 | 14.108 ° | 6.27270 Å | 33.3662 | 84.1151 | 12.2% |
| 9 | 14.994 ° | 5.90386 Å | 200.885 | 250.019 | 73.7% |
| 10 | 16.052 ° | 5.51694 Å | 24.5559 | 70.5282 | 9.0% |
| 11 | 16.776 ° | 5.28053 Å | 161.823 | 208.012 | 59.4% |
| 12 | 17.326 ° | 5.11426 Å | 26.1945 | 73.0762 | 9.6% |
| 13 | 17.967 ° | 4.93310 Å | 55.3122 | 102.242 | 20.3% |
| 14 | 18.924 ° | 4.68561 Å | 61.2474 | 109.116 | 22.5% |
| 15 | 19.367 ° | 4.57950 Å | 50.4790 | 99.5727 | 18.5% |
| 16 | 19.724 ° | 4.49742 Å | 190.946 | 240.452 | 70.0% |
| 17 | 20.386 ° | 4.35290 Å | 272.608 | 322.232 | 100.0% |
| 18 | 20.701 ° | 4.28722 Å | 46.5768 | 95.8588 | 17.1% |
| 19 | 22.419 ° | 3.96246 Å | 62.8885 | 113.536 | 23.1% |
| 20 | 22.541 ° | 3.94138 Å | 51.5345 | 102.501 | 18.9% |
| 21 | 22.948 ° | 3.87231 Å | 72.7512 | 124.356 | 26.7% |
| 22 | 23.246 ° | 3.82344 Å | 95.2536 | 146.901 | 34.9% |
| 23 | 23.639 ° | 3.76077 Å | 50.7536 | 102.312 | 18.6% |
| 24 | 24.145 ° | 3.68295 Å | 48.1779 | 100.838 | 17.7% |
| 25 | 24.850 ° | 3.58011 Å | 104.339 | 158.193 | 38.3% |
| 26 | 26.119 ° | 3.40892 Å | 37.0381 | 92.1590 | 13.6% |
| 27 | 26.486 ° | 3.36252 Å | 42.6422 | 98.5454 | 15.6% |
| 28 | 27.197 ° | 3.27626 Å | 29.0067 | 84.8808 | 10.6% |
| 29 | 28.101 ° | 3.17287 Å | 19.9068 | 74.8151 | 7.3% |
| 30 | 28.607 ° | 3.11791 Å | 52.0044 | 106.816 | 19.1% |
| 31 | 30.157 ° | 2.96110 Å | 19.6713 | 73.1954 | 7.2% |
| 32 | 31.376 ° | 2.84880 Å | 27.2653 | 78.0214 | 10.0% |
| 33 | 33.555 ° | 2.66855 Å | 27.7051 | 75.4432 | 10.2% |
| 34 | 36.073 ° | 2.48788 Å | 17.0289 | 67.3805 | 6.2% |
| 35 | 37.752 ° | 2.38097 Å | 28.0016 | 72.7415 | 10.3% |

### 2. Polymorph Characterization

Choline salt Form A was a hydrate. Choline salt Form A had high crystallinity. DSC showed that it started dehydration at around 21°C, and had a melting point Tₒₙₛₑₜ of 137.8°C and a melting enthalpy of approximately 50 J/g. TGA showed that it had a weight loss of 2.6% at around 80°C, and approximately 0.1% weight loss between 80°C and 130°C. ¹H-NMR showed that it had a stoichiometric ratio of free acid:choline of 1:1.0, with no residual solvent detected in the sample. Water activity competition experiment showed that choline salt Form A transformed to choline salt Form B at 5°C when a.w. ≤ 0.6 and at 25°C when a.w. ≤ 0.3.

Choline salt Form B was an anhydrous form. Choline salt Form B had high crystallinity. DSC showed that it had a melting point of 153.3°C with a melting enthalpy of approximately 62 J/g. TGA showed that it had a weight loss of approximately 0.7% at around 140°C. ¹H-NMR showed that it had a stoichiometric ratio of free acid:choline of 1:1.0, with no residual solvent detected in the sample. Choline salt Form B was the most stable crystal form discovered so far. Water activity competition experiment showed that choline salt Form B was thermodynamically more stable than choline salt Form A at 5°C when a.w. ≤ 0.6 and at 25°C when a.w. ≤ 0.3.

Choline salt Form C was an anhydrous form. Choline salt Form C had high crystallinity. DSC showed that it had a melting point of 155.6°C with a melting enthalpy of approximately 59 J/g. ¹H-NMR showed that it had a stoichiometric ratio of free acid:choline of 1:1.0, with no residual solvent detected in the sample. Choline salt Form C transformed to choline salt Form B after 2 days under ambient conditions (approximately 20 to 25 °C, 60% to 75% RH).

**Table 4-1: Summary of polymorph characterization**

| | Choline salt Form A | Choline salt Form B | Choline salt Form C |
|---|---|---|---|
| Crystallinity (XRPD) | High crystallinity | High crystallinity | High crystallinity |
| Melting point (DSC, °C) | Dehydration: about 21°C; Melting point Tₒₙₛₑₜ: 137.8°C | Melting point Tₒₙₛₑₜ: 153.3°C | Melting point Tₒₙₛₑₜ: 155.6°C |
| Melting enthalpy (DSC, J/g) | About 50J/g | About 62J/g | About 59J/g |
| Thermal weight loss (TGA) | 2.6% @ 80°C; 0.1% @ 80°C to 130°C | 0.7% @ 140°C | // |
| Stoichiometric ratio (¹H-NMR) | 1:1.0 | 1:1.0 | 1:1.0 |
| Residual solvent (¹H-NMR) | No residual solvent detected | No residual solvent detected | No residual solvent detected |

| | | | |
|---|---|---|---|
| Note: "//": Characterization not performed. | | | |

Therein, in order to investigate the critical water activity between T-5224 choline salt Form A and Form B, water activity experiments were conducted in an ethanol/water system at 5°C and 25°C. The method was as follows.

Approximately 2 mg of choline salt Form A, 2 mg of choline salt Form B, and 1 mg of choline salt Form B with additional diffraction peaks were weighed and placed in 0.1 mL of the corresponding saturated ethanol/water solutions, respectively. The resulting suspensions were suspended at 5°C and 25°C for 5 days. The resulting suspensions were then centrifuged, and the obtained solids were analyzed by XRPD. The results are shown in Table 4-2 below.

Therein, the choline salt Form B with additional diffraction peaks was prepared as follows:
Approximately 10 to 20 mg of T-5224 choline salt Form B was weighed and dissolved completely in the minimum volume of solvent (ethanol, 2.0 mL) at ambient temperature (approximately 20 to 25 °C). The resulting solution or thin suspension was filtered through a 0.45 µm nylon membrane syringe filter to obtain a clear solution. 4 to 8 times the volume of anti-solvent (n-heptane, 16 mL) was slowly added to the clear solution. The resulting suspension was centrifugally filtered using a 0.45 µm nylon membrane centrifuge tube at 14,000 rpm, and the obtained solid was characterized by XRPD.

**Table 4-2: Water activity experiments**

| **Starting crystal forms: choline salt Form A and choline salt Form B** | | | |
|---|---|---|---|
| **Exp. No.** | **Solvent** | **XRPD after 5 days at 5°C** | **XRPD after 5 days at 25°C** |
| **Starting crystal forms: choline salt Form A and choline salt Form B** | | | |
| AW1 | Water (a.w.=1)* | T-5224 Form D, gel-like sample | T-5224 Form D, gel-like sample |
| AW9 | Ethanol/water (80:20,v:v) (a.w.=0.7)* | T-5224 Form A + Form C | // |
| AW2 | Ethanol/water (86:14,v:v) (a.w.=0.6)* | Choline salt Form B | T-5224 Form C with additional diffraction peaks (possibly from T-5224 Form A) |
| AW3 | Ethanol/water (90:10,v:v) (a.w.=0.5)* | Choline salt Form B | T-5224 Form C |
| AW4 | Ethanol/water (93:7,v:v) (a.w.=0.4)* | Choline salt Form B | T-5224 Form C + Choline salt Form B |
| AW5 | Ethanol/water (95:5,v:v) (a.w.=0.3)* | Choline salt Form B | Choline salt Form B |
| AW6 | Ethanol/water (97:3,v:v) (a.w.=0.2)* | Choline salt Form B | Choline salt Form B |
| AW7 | Ethanol/water (99:1,v:v) (a.w.=0.1)* | Choline salt Form B | Choline salt Form B |
| AW8 | Ethanol (a.w.=0)* | Choline salt Form B | Choline salt Form B |

| | | | |
|---|---|---|---|
| Note: "//": not performed; "*": Water activity (a.w.) calculated by the UNIFAC method. | | | |

### 3. Evaluation of Properties of T-5224 Choline Salt Form B

T-5224 choline salt Form B was the most stable choline salt crystal form discovered so far. Therefore, systematic studies on the stability, solubility, and mechanical properties of T-5224 choline salt Form B were conducted.

### 3.1 Solid Stability

In this study, the solid stability of choline salt Form B was evaluated under accelerated conditions: open conditions at 25°C/92.5% RH and closed conditions at 60°C for 1 week.

Open containers containing T-5224 choline salt Form B were placed at 25°C/92.5% RH for 1 week. Closed containers containing T-5224 choline salt Form B were placed at 60°C for 1 week. Samples from these conditions were analyzed by XRPD and HPLC, and color changes were observed.

**Table 4-3: Solid stability**

| | | |
|---|---|---|
| Initial Purity | 98.8% | |

| | Purity | Color |
|---|---|---|
| Solid, 25°C/92.5%RH, open containers, 1 week | | |
| Solid (HPLC) | 98.8% | A |
| Solid (XRPD) | Choline salt Form B | |

| Solid, 60°C, closed containers, 1 week | | |
|---|---|---|
| Solid (HPLC) | 98.8% | A |
| Initial Purity | 98.8% | |

| | Purity | Color |
|---|---|---|
| Solid (XRPD) | Choline salt Form B | |

| | | |
|---|---|---|
| Note: A: No color change; B: Slight color change; C: Moderate color change; D: Severe color change. | | |

Choline salt Form B exhibited good physical and chemical stability under the accelerated stability conditions mentioned above.

### 3.2 Solubility

In this study, the solubility of the starting material T-5224 Form A and choline salt Form B was evaluated in water, pH 1.6 FaSSGF solution, pH 6.5 FaSSIF solution, and pH 5.0 FeSSIF solution at 37°C for 2 and 24 hours. The residual solids from the solubility experiments were characterized by XRPD.

10.0 mg of T-5224 Form A and 12.5 mg of choline salt Form B were precisely weighed and placed into 8 mL glass vials. 5 mL of the respective solution medium was added. The mass of salt weighed corresponded to 10 mg of T-5224 Form A. The resulting suspensions were stirred at 37°C at 400 rpm, and samples were taken at 2 and 24 hours. The samples were centrifuged at 14,000 rpm for 5 minutes at 37°C. The supernatant concentration was determined by HPLC, and the pH of the supernatant was measured using a pH meter.

**Table 4-4: Solubility**

| Solubility at 37°C; Target concentration: 2mg/mL (as T-5224); Equilibration: 24 hours; LOQ: 0.5µg/mL | | | | | | |
|---|---|---|---|---|---|---|
| | Physical form | | | | | |
| | T-5224 Form A | | | Choline salt Form B | | |
| Solution medium | Solubility | | | Solubility | | |
| | 2h | 24h (pH) | | 2h | 24h (pH) | |
| Water | 7.8µg/mL | 8.9µg/mL (6.3) | | >2mg/mL | >2mg/mL (7.7) | |
| FaSSGF, pH 1.6 | <LOQ | <LOQ (1.6) | | <LOQ | <LOQ (1.6) | |
| FaSSIF-v1, pH 6.5 | 49.6µg/mL | 33.1µg/mL (6.4) | | 52.5µg/mL | 44.1µg/mL (6.7) | |
| FeSSIF-v1, pH 5.0 | 23.3µg/mL | 20.9µg/mL (4.8) | | 20.6µg/mL | 30.2µg/mL (5.0) | |

The solubility of T-5224 Form A and choline salt Form B was pH-dependent. The 24-hour solubility of choline salt Form B in pH 6.5 FaSSIF-v1 and pH 5.0 FeSSIF-v1 (30 to 45 µg/mL) was slightly higher than that of T-5224 Form A (20 to 35 µg/mL). In pH 1.6 FaSSGF, the solubility of both T-5224 Form A and choline salt Form B was below the limit of quantitation (LOQ).

The solubility of choline salt Form B in water (>2 mg/mL) was much higher than that of T-5224 Form A (8 to 9 µg/mL).

### 3.3 Mechanical Properties

In this study, the mechanical properties of choline salt Form B were evaluated through simulated tableting, grinding, and granulation experiments.

### 3.3.1 Simulated Tableting Experiment

Approximately 10 mg of T-5224 choline salt Form B sample was weighed and compressed at pressures of 2 MPa, 5 MPa, and 10 MPa for 5 minutes. The crystal form transition and changes in crystallinity were studied by XRPD.

**Table 4-5: Simulated tableting experiment**

| Exp. No. | Pressure | XRPD | Remarks |
|---|---|---|---|
| CSE1 | 2MPa | Choline salt Form B | No significant change in crystallinity |
| CSE2 | 5MPa | Choline salt Form B | Slight decrease in crystallinity |
| CSE3 | 10MPa | Choline salt Form B | Slight decrease in crystallinity |

### 3.3.2 Simulated Dry Grinding Experiment

Approximately 10 mg of T-5224 choline salt Form B sample was weighed and manually ground for 5 minutes. The crystal form transition and changes in crystallinity were studied by XRPD.

**Table 4-6: Simulated dry grinding experiment**

| Exp. No. | Grinding time | XRPD | Remarks |
|---|---|---|---|
| GSE1 | 5 min | Choline salt Form B | No significant change in crystallinity |

### 3.3.3 Simulated Wet Granulation Experiment

Approximately 10 mg of T-5224 choline salt Form B sample was weighed, and ethanol was added dropwise until the sample was thoroughly wetted. The wetted sample was gently ground with a pestle and then dried under ambient conditions for 10 minutes. The crystal form transition and changes in crystallinity were studied by XRPD.

**Table 4-7: Simulated wet granulation experiment**

| Exp. No. | Granulation solvent | XRPD | Remarks |
|---|---|---|---|
| GNSE1 | Ethanol | Choline salt Form B | No significant change in crystallinity |

Choline salt Form B showed good tolerance to tableting (<2 MPa), dry grinding, and wet grinding, with no crystal form transition and no significant decrease in crystallinity. When the tableting pressure was increased to 5 to 10 MPa, choline salt Form B showed no crystal form transition, and its crystallinity only slightly decreased.

Based on the above research results, T-5224 choline salt Form B was the most stable crystal form obtained so far. It exhibited good chemical stability, physical stability, and mechanical force tolerance. Regarding solubility, the 24-hour solubility of choline salt Form B in water, pH 6.5 FaSSIF solution, and pH 5.0 FeSSIF solution was higher than that of the initial material T-5224 Form A. Therefore, T-5224 choline salt Form B was selected as the preferred crystal form for subsequent pharmacokinetic studies.

### Example 5: Pharmacokinetic Study and Comparison of T-5224 free acid Form A (i.e., the aforementioned T-5224 Form A) and T-5224 Choline Salt Crystal Form B

### 1. Administration Method

| Animal experiments for pharmacokinetics | | | | | | | | |
|---|---|---|---|---|---|---|---|---|
| Group | Animal Info | Fasted/ Fed | Compound | Dose (mg/kg) | Dose Volume (ml/kg) | Formulation Conc. (mg/ml) | Sample type | Detection time points (hours) |
| Intravenous injection | 3 Balb/c mice, male | Fasted | T-5224 Form A | 2 | 10 | 0.20 | Plasma | 0.0833, 0.25, 0.5, 1, 2, 4, 6, 8, 24 |
| Oral administration | 3 Balb/c mice, male | Fasted | T-5224 Form A | 12 | 10 | 1.20 | Plasma | 0.25, 0.5, 1,2, 4, 6, 8, 24 |
| Oral administration | 3 Balb/c mice, male | Fasted | Choline salt Form B | 12 | 10 | 1.20 | Plasma | 0.25, 0.5, 1, 2, 4, 6, 8, 24 |

### 2. Formulation Preparation Method

| Group | Compound | Compound mass (mg) | Salt type | Solvent composition | Volume (mL) | Suspension time (min) | Sonication time (min) | Formulation status |
|---|---|---|---|---|---|---|---|---|
| Intravenous injection | T-5224 Form A | 0.57 | 1 | 5% DMSO + 95% (20% Captisol) | 2.82 | 2 | 1 | Clear solution |
| Oral administration | T-5224 Form A | 1.44 | 1 | 0.5% PVP | 1.19 | 3 | 2 | Suspension |
| Oral administration | Choline salt Form B | 1.45 | 1 | 0.5% PVP | 1.20 | 3 | NA | Clear solution |

### 3. Detection Method

LC-MS/MS

### 1. Instrument

API-4000 Qtrap-Shimadzu Controller-CBM20A

### 2. MS Conditions

Positive ion mode:

| Compound name | Parent(m/z) | Daughter(m/z) | Tube lens | CE |
|---|---|---|---|---|
| Tolbutamide | 271.2 | 155 | 73 | 23 |
| free | 518.4 | 148 | 107 | 44 |

### 3. LC Conditions

MP-A: 0.1% formic acid
MP-B: MeOH
HPLC column: Waters XBridge C18, 3.5 um, 2.1*50 mm
Gradient:

| Step | Total Time (min) | Flow rate (ml/min) | A% | B% |
|---|---|---|---|---|
| 1 | 0.01 | 0.45 | 98 | 2 |
| 2 | 0.30 | 0.45 | 98 | 2 |
| 3 | 1.20 | 0.45 | 2 | 98 |
| 4 | 2.50 | 0.45 | 2 | 98 |
| 5 | 2.51 | 0.45 | 98 | 2 |
| 6 | 3.50 | 0.45 | 98 | 2 |

### 4. Sample Preparation:

a) 20 µl of plasma sample was transferred to a 96-well plate.
b) 250 µl of acetonitrile (containing internal standard) was added for protein precipitation.
c) Centrifugation was performed at 4000 rpm for 20 min at 4 °C.
d) 180 µl of supernatant was transferred to a new 96-well plate and mixed with 180 µl of water.
e) 5 µl was pipetted for LC-MS/MS detection.

### 4. Experimental Results

| T-5224 free acid Form A PK parameters and plasma concentration detection | | | | | | | |
|---|---|---|---|---|---|---|---|
| Time for intravenous injection (hr) | M301 | M302 | M303 | Mean | | S.D. | CV (%) |
| 0.0833 | 1850 | 2300 | 2090 | 2080 | | 225 | 10.8 |
| 0.25 | 208 | 263 | 637 | 369 | ± | 233 | 63.2 |
| 0.5 | 112 | 151 | 230 | 164 | ± | 60.1 | 36.6 |
| 1 | 58.0 | 85.6 | 125 | 89.5 | ± | 33.7 | 37.6 |
| 2 | 22.9 | 49.8 | 44.7 | 39.1 | ± | 14.3 | 36.5 |
| 4 | 18.1 | 29.3 | 21.5 | 23.0 | ± | 5.74 | 25.0 |
| 6 | 9.80 | 9.83 | 13.8 | 11.1 | ± | 2.30 | 20.6 |
| 8 | BLQ | BLQ | BLQ | n.d. | ± | n.d. | n.d. |
| 24 | BLQ | BLQ | BLQ | n.d. | ± | n.d. | n.d. |
| C0(ng/ml) | 5514 | 6797 | 3784 | 5365 | ± | 1512 | 28.2 |
| T1/2 (hr) | 3.27 | 1.68 | 2.36 | 2.44 | ± | 0.80 | 32.6 |
| AUC₀₋ₜ (ng·hr/mL) | 670 | 889 | 855 | 805 | ± | 118 | 14.7 |
| AUC_{0-∞} (ng·hr/mL) | 716 | 913 | 902 | 844 | ± | 111 | 13.1 |
| Vz (L/Kg) | 13.16 | 5.31 | 7.54 | 8.67 | ± | 4.04 | 46.6 |
| Cl (mL/min/kg) | 46.5 | 36.5 | 36.9 | 40.0 | ± | 5.67 | 14.2 |
| Vss (L/Kg) | 3.36 | 1.86 | 2.51 | 2.58 | ± | 0.75 | 29.3 |
| MRT(hr) | 0.55 | 0.64 | 0.68 | 0.62 | ± | 0.07 | 10.9 |

| T-5224 free acid Form A PK parameters and plasma concentration detection | | | | | | | |
|---|---|---|---|---|---|---|---|
| Time for oral administration (hr) | M304 | M305 | M306 | Mean | | S.D. | CV (%) |
| 0.25 | 562 | 751 | 920 | 744 | ± | 179 | 24.1 |
| 0.5 | 462 | 328 | 519 | 436 | + | 98.1 | 22.5 |
| 1 | 298 | 343 | 263 | 301 | + | 40.1 | 13.3 |
| 2 | 294 | 55.9 | 126 | 159 | ± | 122 | 77.1 |
| 4 | 30.6 | 17.0 | 22.2 | 23.3 | + | 6.86 | 29.5 |
| 6 | 12.5 | 8.17 | 8.46 | 9.71 | + | 2.42 | 24.9 |
| 8 | BLQ | BLQ | BLQ | n.d. | + | n.d. | n.d. |
| 24 | BLQ | BLQ | BLQ | n.d. | + | n.d. | n.d. |
| T1/2 (hr) | 1.00 | 1.44 | 0.98 | 1.14 | ± | 0.26 | 22.7 |
| Tmax (hr) | 0.25 | 0.25 | 0.25 | 0.25 | ± | 0.00 | 0.00 |
| Cmax (ng/mL) | 562 | 751 | 920 | 744 | ± | 179 | 24.1 |
| AUC₀₋ₜ (ng·hr/mL) | 1052 | 694 | 864 | 870 | ± | 179 | 20.6 |
| AUC_{0-∞} (ng·hr/mL) | 1070 | 711 | 876 | 886 | ± | 180 | 20.3 |
| Vz -F(L/Kg) | 16.2 | 35.1 | 19.5 | 23.6 | ± | 10.1 | 42.8 |
| Cl -F(mL/min/kg) | 187 | 281 | 228 | 232 | ± | 47.3 | 20.4 |
| MRT(hr) | 1.47 | 1.04 | 1.11 | 1.21 | ± | 0.23 | 19.3 |
| Bioavailability (%) | | | | **17.5%** | | | |

| T-5224 choline salt Form B PK parameters and plasma concentration detection | | | | | | | |
|---|---|---|---|---|---|---|---|
| Time for oral administration (hr) | M307 | M308 | M309 | Mean | | S.D. | CV (%) |
| 0.25 | 5540 | 4330 | 5390 | 5087 | ± | 660 | 13.0 |
| 0.5 | 1640 | 1510 | 2600 | 1917 | ± | 595 | 31.1 |
| 1 | 457 | 574 | 573 | 535 | ± | 67.3 | 12.6 |
| 2 | 355 | 159 | 148 | 221 | ± | 116 | 52.8 |
| 4 | 27.0 | 47.0 | 27.9 | 34.0 | ± | 11.3 | 33.3 |
| 6 | 5.85 | 4.58 | 8.58 | 6.34 | ± | 2.04 | 32.3 |
| 8 | BLQ | BLQ | BLQ | n.d. | ± | n.d. | n.d. |
| 24 | BLQ | BLQ | BLQ | n.d. | ± | n.d. | n.d. |
| T1/2 (hr) | 0.70 | 0.75 | 0.97 | 0.81 | ± | 0.15 | 18.1 |
| Tmax (hr) | 0.25 | 0.25 | 0.25 | 0.25 | ± | 0.00 | 0.00 |
| Cmax (ng/mL) | 5540 | 4330 | 5390 | 5087 | ± | 660 | 13.0 |
| AUC₀₋ₜ (ng·hr/mL) | 2935 | 2416 | 3039 | 2797 | ± | 333 | 11.9 |
| AUC_{0-∞} (ng·hr/mL) | 2941 | 2421 | 3051 | 2804 | ± | 336 | 12.0 |
| Vz -F(L/Kg) | 4.10 | 5.39 | 5.53 | 5.01 | ± | 0.79 | 15.7 |
| Cl -F(mL/min/kg) | 68.0 | 82.6 | 65.6 | 72.1 | ± | 9.21 | 12.8 |
| MRT(hr) | 0.79 | 0.77 | 0.65 | 0.74 | ± | 0.08 | 10.3 |
| Bioavailability (%) | | | | **55.4%** | | | |

### 5. Experimental Conclusion

From the above results, it can be seen that compared to the control T-5224 Form A, the choline salt Form B of the present application exhibited significantly improved bioavailability (55.4% vs 17.5%), representing an increase of at least 2-fold and demonstrating a significant advantage.

### Example 6: Comparison of Therapeutic Efficacy in Animal Models using T-5224 free acid Form A (i.e., the aforementioned T-5224 Form A) and T-5224 choline salt Crystal Form B

Materials and reagents, animal experiment guidelines, and the heart failure with preserved ejection fraction model induction were the same as in Example A.

The administration method was as follows:
After successful induction of the animal model, mice in the model treatment groups were treated with T-5224 free acid Form A and T-5224 choline salt Form B, while the model control group was treated with the drug-free vehicle as a control. Throughout the induction period, mice fed a normal diet and water were used as a negative control. After the mice were raised to 5 to 8 weeks of age, drug treatment was initiated following echocardiography and Doppler imaging confirmation of successful model establishment. For the treatment groups, T-5224 free acid Form A was administered at a dose of 12 mg/kg per administration based on mouse body weight. Specifically, the administration was performed every other day. And at each time, 0.54 mg of the compound was dissolved in 100 µL of 0.5% PVP solution (the actual preparation was scaled according to the number of mice; for example, for 16 mice, 9 mg of the drug was dissolved in 1.7 mL of solution, and administered at 100 µL per mouse). T-5224 choline salt Form B was administered at a dose of 3 mg/kg per administration based on mouse body weight. Specifically, the administration was performed every other day, and at each time, 0.14 mg of the compound was dissolved in 100 µL of 0.5% PVP solution (the actual preparation was scaled according to the number of mice; for example, for 16 mice, 2.2 mg of the drug was dissolved in 1.7 mL of solution, and administered at 100 µL per mouse). The administration started upon successful model induction and continued for 7 weeks. The control group received an equal volume of 0.5% PVP solution, with all other treatments being identical.

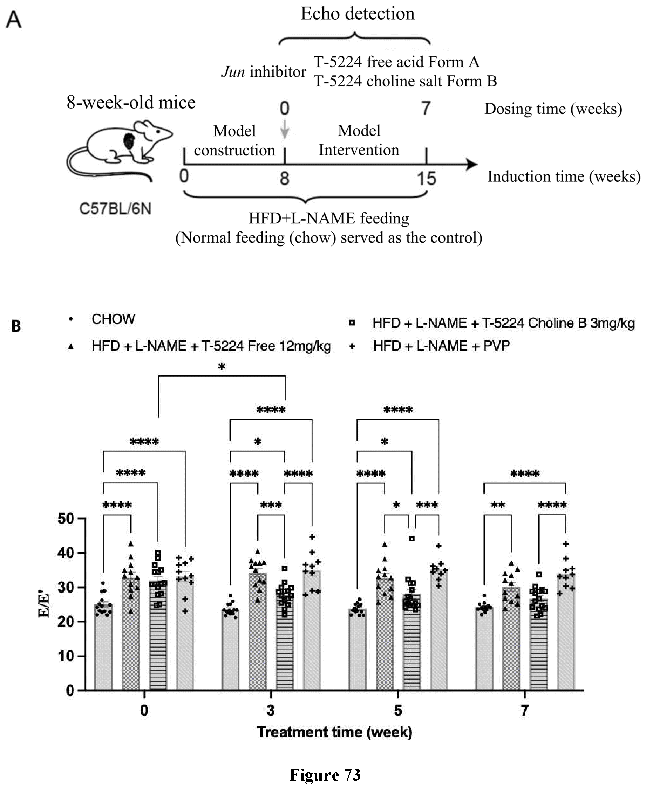

The experimental results are shown in Figure 73 (wherein, week 0 on the horizontal axis represents the day when administration started after successful model construction, i.e., the starting point of treatment). It can be clearly observed from Figure 73 that as early as 3 weeks after administration, the diastolic function of mice treated with T-5224 choline salt Form B (3 mg/kg) was significantly improved (E/E' significantly decreased), whereas the diastolic function of mice treated with the positive control T-5224 free acid Form A (12 mg/kg) had not yet shown significant changes. It is evident that compared to the positive control T-5224 free acid Form A, T-5224 choline salt Form B could inhibit the occurrence and development of the disease earlier and at a significantly lower dose, demonstrating clear advantages and broad application prospects. Therefore, the salt forms and crystal forms thereof of the present invention (especially T-5224 choline salt Form B) have a significant therapeutic effect on HFpEF at a relatively low dose, with a rapid onset of action.

## Claims

1. A salt of a compound represented by Formula I, wherein the salt is a sodium salt, potassium salt, calcium salt, tert-butylamine salt, ammonium salt, choline salt, L-lysine salt, diethylamine salt, dimethylethanolamine salt, betaine salt, or ethylenediamine salt of the compound represented by Formula I,

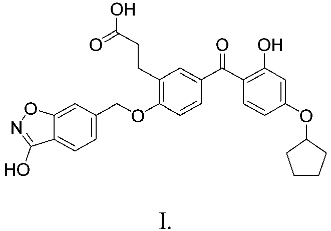

2. The salt according to claim 1, wherein the salt is a sodium salt, potassium salt, tert-butylamine salt, choline salt, L-lysine salt, diethylamine salt, dimethylethanolamine salt, betaine salt, or ethylenediamine salt of the compound represented by Formula I.

3. The salt according to claim 1 or 2, wherein the salt is a choline salt, diethylamine salt, or betaine salt of the compound represented by Formula I.

4. The salt according to any one of claims 1-3, wherein the salt is a choline salt or betaine salt of the compound represented by Formula I.

5. The salt according to any one of claims 1 to 4, wherein the salt is a choline salt of the compound represented by Formula I.

6. The salt according to any one of claims 1 to 5, wherein the salt further has one or more technical features selected from the group consisting of following items (1) to (20):
(1) the sodium salt of the compound represented by Formula I is in crystal Form A, wherein the crystal Form A of the sodium salt of the compound represented by Formula I has an X-ray powder diffraction pattern substantially the same as that shown in Figure 4;
(2) the sodium salt of the compound represented by Formula I is in crystal Form B, wherein the crystal Form B of the sodium salt of the compound represented by Formula I has an X-ray powder diffraction pattern substantially the same as that shown in Figure 6;
(3) the potassium salt of the compound represented by Formula I is in crystal Form A, wherein the crystal Form A of the potassium salt of the compound represented by Formula I has an X-ray powder diffraction pattern substantially the same as that shown in Figure 10;
(4) the potassium salt of the compound represented by Formula I is in crystal Form B, wherein the crystal Form B of the potassium salt of the compound represented by Formula I has an X-ray powder diffraction pattern substantially the same as that shown in Figure 12;
(5) the potassium salt of the compound represented by Formula I is in crystal Form C, wherein the crystal Form C of the potassium salt of the compound represented by Formula I has an X-ray powder diffraction pattern substantially the same as that shown in Figure 16;
(6) the potassium salt of the compound represented by Formula I is in crystal Form D, wherein the crystal Form D of the potassium salt of the compound represented by Formula I has an X-ray powder diffraction pattern substantially the same as that shown in Figure 18;
(7) the calcium salt of the compound represented by Formula I is in crystal Form A, wherein the crystal Form A of the calcium salt of the compound represented by Formula I has an X-ray powder diffraction pattern substantially the same as that shown in Figure 19;
(8) the tert-butylamine salt of the compound represented by Formula I is in crystal Form A, wherein the crystal Form A of the tert-butylamine salt of the compound represented by Formula I has an X-ray powder diffraction pattern substantially the same as that shown in Figure 20;
(9) the tert-butylamine salt of the compound represented by Formula I is in crystal Form B, wherein the crystal Form B of the tert-butylamine salt of the compound represented by Formula I has an X-ray powder diffraction pattern substantially the same as that shown in Figure 22;
(10) the ammonium salt of the compound represented by Formula I is in crystal Form A, wherein the crystal Form A of the ammonium salt of the compound represented by Formula I has an X-ray powder diffraction pattern substantially the same as that shown in Figure 26;
(11) the ammonium salt of the compound represented by Formula I is in crystal Form B, wherein the crystal Form B of the ammonium salt of the compound represented by Formula I has an X-ray powder diffraction pattern substantially the same as that shown in Figure 27;
(12) the L-lysine salt of the compound represented by Formula I is in crystal Form A, wherein the crystal Form A of the L-lysine salt of the compound represented by Formula I has an X-ray powder diffraction pattern substantially the same as that shown in Figure 32;
(13) the diethylamine salt of the compound represented by Formula I is in crystal Form A, wherein the crystal Form A of the diethylamine salt of the compound represented by Formula I has an X-ray powder diffraction pattern substantially the same as that shown in Figure 36;
(14) the diethylamine salt of the compound represented by Formula I is in crystal Form B, wherein the crystal Form B of the diethylamine salt of the compound represented by Formula I has an X-ray powder diffraction pattern substantially the same as that shown in Figure 40;
(15) the diethylamine salt of the compound represented by Formula I is in crystal Form C, wherein the crystal Form C of the diethylamine salt of the compound represented by Formula I has characteristic peaks in its X-ray powder diffraction pattern at 2θ angles of 4.7±0.2°, 6.4±0.2°, 9.4±0.2°, 13.8±0.2°, 15.0±0.2°, 16.8±0.2°, 19.7±0.2°, and 20.4±0.2°;
preferably, the crystal Form C of the diethylamine salt of the compound represented by Formula I has characteristic peaks in its X-ray powder diffraction pattern at 2θ angles of 4.7±0.2°, 6.4±0.2°, 9.4±0.2°, 13.1±0.2°, 13.8±0.2°, 14.1±0.2°, 15.0±0.2°, 16.8±0.2°, 18.0±0.2°, 18.9±0.2°, 19.4±0.2°, 19.7±0.2°, 20.4±0.2°, 23.0±0.2°, 23.3±0.2°, and 24.9±0.2°;
more preferably, the crystal Form C of the diethylamine salt of the compound represented by Formula I has an X-ray powder diffraction pattern with 2θ, d, and peak intensity values as shown in Table C;
most preferably, the crystal Form C of the diethylamine salt of the compound represented by Formula I has an X-ray powder diffraction pattern substantially the same as that shown in Figure 62;
(16) the dimethylethanolamine salt of the compound represented by Formula I is in crystal Form A, wherein the crystal Form A of the dimethylethanolamine salt of the compound represented by Formula I has an X-ray powder diffraction pattern substantially the same as that shown in Figure 44;
(17) the betaine salt of the compound represented by Formula I is in crystal Form A, wherein the crystal Form A of the betaine salt of the compound represented by Formula I has characteristic peaks in its X-ray powder diffraction pattern at 2θ angles of 8.7±0.2°, 11.8±0.2°, 15.4±0.2°, 16.3±0.2°, 19.6±0.2°, 21.4±0.2°, and 23.4±0.2°;
preferably, the crystal Form A of the betaine salt of the compound represented by Formula I has characteristic peaks in its X-ray powder diffraction pattern at 2θ angles of 8.1±0.2°, 8.7±0.2°, 11.8±0.2°, 15.4±0.2°, 16.3±0.2°, 18.4±0.2°, 19.6±0.2°, 20.4±0.2°, 21.4±0.2°, 22.3±0.2°, 23.4±0.2°, 24.2±0.2°, and 25.9±0.2°;
more preferably, the crystal Form A of the betaine salt of the compound represented by Formula I has an X-ray powder diffraction pattern with 2θ, d, and peak intensity values as shown in Table A;
most preferably, the crystal Form A of the betaine salt of the compound represented by Formula I has an X-ray powder diffraction pattern substantially the same as that shown in Figure 48;
(18) the ethylenediamine salt of the compound represented by Formula I is in crystal Form A, wherein the crystal Form A of the ethylenediamine salt of the compound represented by Formula I has an X-ray powder diffraction pattern substantially the same as that shown in Figure 52;
(19) the ethylenediamine salt of the compound represented by Formula I is in crystal Form B, wherein the crystal Form B of the ethylenediamine salt of the compound represented by Formula I has an X-ray powder diffraction pattern substantially the same as that shown in Figure 54;
(20) the choline salt of the compound represented by Formula I is in crystal Form C, wherein the crystal Form C of the choline salt of the compound represented by Formula I has characteristic peaks in its X-ray powder diffraction pattern at 2θ angles of 4.7±0.2°, 6.4±0.2°, 9.4±0.2°, 15.0±0.2°, 16.8±0.2°, 19.7±0.2°, and 20.4±0.2°;
preferably, the crystal Form C of the choline salt of the compound represented by Formula I has characteristic peaks in its X-ray powder diffraction pattern at 2θ angles of 4.7±0.2°, 6.4±0.2°, 9.4±0.2°, 13.1±0.2°, 13.8±0.2°, 15.0±0.2°, 16.8±0.2°, 18.9±0.2°, 19.7±0.2°, 20.4±0.2°, 22.4±0.2°, 22.9±0.2°, 23.2±0.2°, and 24.9±0.2°;
more preferably, the crystal Form C of the choline salt of the compound represented by Formula I has an X-ray powder diffraction pattern with 2θ, d, and peak intensity values as shown in Table E;
most preferably, the crystal Form C of the choline salt of the compound represented by Formula I has an X-ray powder diffraction pattern substantially the same as that shown in Figure 70.

7. The salt according to any one of claims 1 to 6, wherein the choline salt of the compound represented by Formula I is in crystal Form A, wherein the crystal Form A of the choline salt of the compound represented by Formula I has characteristic peaks in its X-ray powder diffraction pattern at 2θ angles of 6.9±0.2°, 14.0±0.2°, 19.7±0.2°, 21.2±0.2°, 22.8±0.2°, 26.5±0.2°, and 27.0±0.2°;
preferably, the crystal Form A of the choline salt of the compound represented by Formula I has characteristic peaks in its X-ray powder diffraction pattern at 2θ angles of 6.9±0.2°, 11.7±0.2°, 14.0±0.2°, 15.0±0.2°, 17.7±0.2°, 18.6±0.2°, 19.1±0.2°, 19.7±0.2°, 20.9±0.2°, 21.2±0.2°, 22.8±0.2°, 26.3±0.2°, 26.5±0.2°, 27.0±0.2°, and 27.6±0.2°;
more preferably, the crystal Form A of the choline salt of the compound represented by Formula I has an X-ray powder diffraction pattern with 2θ, d, and peak intensity values as shown in Table B;
most preferably, the crystal Form A of the choline salt of the compound represented by Formula I has an X-ray powder diffraction pattern substantially the same as that shown in Figure 28.

8. The salt according to any one of claims 1 to 7, wherein the choline salt of the compound represented by Formula I is in crystal Form B, wherein the crystal Form B of the choline salt of the compound represented by Formula I has characteristic peaks in its X-ray powder diffraction pattern at 2θ angles of 8.9±0.2°, 17.0±0.2°, 17.4±0.2°, 19.5±0.2°, 20.4±0.2°, 22.2±0.2°, 22.7±0.2°, and 27.2±0.2°;
preferably, the crystal Form B of the choline salt of the compound represented by Formula I has characteristic peaks in its X-ray powder diffraction pattern at 2θ angles of 8.9±0.2°, 16.2±0.2°, 17.0±0.2°, 17.4±0.2°, 19.5±0.2°, 19.9±0.2°, 20.4±0.2°, 20.9±0.2°, 21.8±0.2°, 22.2±0.2°, 22.5±0.2°, 22.7±0.2°, 26.5±0.2°, 27.2±0.2°, and 27.5±0.2°;
more preferably, the crystal Form B of the choline salt of the compound represented by Formula I has an X-ray powder diffraction pattern with 2θ, d, and peak intensity values as shown in Table D;
most preferably, the crystal Form B of the choline salt of the compound represented by Formula I has an X-ray powder diffraction pattern substantially the same as that shown in Figure 66.

9. A pharmaceutical composition, comprising the salt according to any one of claims 1 to 8, and optionally further comprising a pharmaceutically acceptable excipient.

10. Use of the salt according to any one of claims 1 to 8 or the pharmaceutical composition according to claim 9 in the manufacture of a medicament for treating and/or preventing heart failure with preserved ejection fraction.
